(19) Europäisches Patentamt
European Patent Office
Office européen des brevets

(11) EP 2 493 462 B1

(12) **EUROPEAN PATENT SPECIFICATION**

(45) Date of publication and mention of the grant of the patent:
**24.07.2013 Bulletin 2013/30**

(21) Application number: **10775793.2**

(22) Date of filing: **29.10.2010**

(51) Int Cl.:
*A61K 31/05* (2006.01)

(86) International application number:
**PCT/EP2010/066544**

(87) International publication number:
**WO 2011/051483 (05.05.2011 Gazette 2011/18)**

# (54) PTEROSTILBENE (PTER) FOR USE IN THE PREVENTION AND/OR TREATMENT OF SKIN DISEASES, DAMAGES OR INJURES

Verwendung von Pterostilben (PTER) als Medikament zur Vorbeugung und/oder Behandlung von Hauterkrankungen, -schädigungen oder Verletzungen oder als Kosmetikmittel

Utilisation de pterostilbene (pter) en tant que médicament pour la prévention et/ou le traitement des maladies de la peau, dommages ou blessures ou en tant que cosmétiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2009 EP 09174717**

(43) Date of publication of application:
**05.09.2012 Bulletin 2012/36**

(73) Proprietor: **Green Molecular, S.L.**
**46980 Valencia (ES)**

(72) Inventors:
- **ESTRELA ARIQUEL, Jose María**
  **E-46980 Valencia (ES)**
- **ASENSI MIRALLES, Miguel A.**
  **E-46980 Valencia (ES)**

(74) Representative: **Illescas, Manuel**
**González-Bueno & Illescas**
**Calle de Recoletos, 13 - 5º Izq.**
**E-28001 Madrid (ES)**

(56) References cited:
**EP-A1- 1 782 802** **WO-A1-03/049713**
**WO-A2-01/43705** **US-A1- 2009 163 580**

- **FERRER P ET AL: "Association between pterostilbene and quercetin inhibits metastatic activity of B16 melanoma", NEOPLASIA, NEOPLASIA PRESS, ANN ARBOR, MI, US LNKD-DOI:10.1593/NEO.04337, vol. 7, no. 1, 1 January 2005 (2005-01-01) , pages 37-47, XP003018478, ISSN: 1522-8002**
- **ORTEGA A ET AL: "INHIBITION OF B16 MELANOMA GROWTH AND METASTATIC ACTIVITY BY COMBINED ADMINISTRATION OF PTEROSTILBENE AND QUERCETIN", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 45, 1 March 2004 (2004-03-01), page 883, XP001536815, ISSN: 0197-016X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention may be encompassed in the pharmaceutical technical field.

### STATE OF THE ART

A high intake of fruits and vegetables is associated with a healthy lifestyle. This association is due to the presence in food of natural antioxidants, mainly polyphenolic compounds. One of the polyphenols that has arisen more interest in the scientific community is resveratrol (3,4',5-trihydroxy-trans-stilbene; RESV), a small polyphenol present in significant amounts in wine, to which potential beneficial effects have been attributed in different chronic diseases (especially cancer, cardiovascular disease, type II diabetes and neurodegenerative diseases). The anticancer properties of RESV were first proposed by Jang et al. (Jang M, Cai L, Udeani GO, Slowing KV, Thomas CF, Beecher CW, Fong HH, Farnsworth NR, Kinghorn AD, Mehta RG, Moon RC, Pezzuto JM. (1997) Science 275(5297):218-20), with effects on the three stages of cancer development (initiation, promotion and progression). The anticancer effects of RESV are very limited by its low bioavailability (Asensi M, Medina I, Ortega A, Carretero J, Bano MC, Obrador E, Estrela JM. (2002) Free adic Biol Metal. 33(3): 387-98*).* Due to that drawback, some other polyphenols with improved bioavailabilty were assayed for cancer treatment. The combination of PTER (3,5-dimethoxy-4'-hydroxy-trans-resveratrol, PTER, a derivative of RESV) and QUER (3,3', 4 ',5,6-pentahidroxiflavona; QUER), two small natural polyphenols, having the following formula:

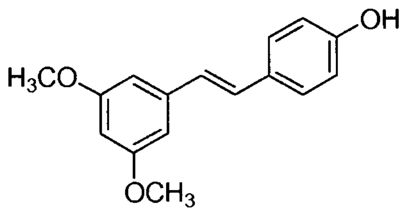

**PTER**

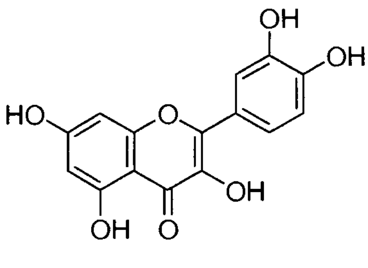

**QUER**

was shown to be effective to inhibit metastatic growth of a highly aggressive murine malignant melanoma (Ferrer P, Asensi M, Segarra R, Ortega A, Benlloch M, Obrador E, Varea MT, Asensio G, Jorda L, Estrela JM. (2005) Neoplasia. 7(1):37-47). Furthermore the association of PTER and QUER, by enhancing the antitumor effects of chemotherapy plus radiotherapy, facilitates the elimination of resistant human colorectal cancer xenografted in immunodeficient mice (Priego S, Feddi F, Ferrer P, Mena S, Benlloch M, Ortega A, Carretero J, Obrador E, Asensi M, Estrela JM. (2008) Mol Cancer Ther. 7(10): 3330-42). PTER and QUER were administered intravenously and/or orally.

Recent epidemiological studies have shown that over 90% of all skin cancers are caused by excessive exposure to sun, especially ultraviolet B (UVB). The incidence of these cancers, both melanoma and non melanoma type, is increasing in a spectacular way (deaths from skin cancer have doubled in the last 20 years).

Therefore, there is a long-felt need in the art for compositions able to prevent and/or treat skin diseases (including skin cancer), injures or damages caused by prolonged exposures to radiation, particularly UVB (ultraviolet B).

However, skin diseases, injures or damages, may be caused, not only by radiation exposure, but also being due to another factors, as immune factors. Most of skin diseases, injures or damages share the presence of inflammatory processes associated to skin pathology. Moreover, skin exposure to radiation or skin inflammatory processes associated

either to said radiation exposure or to other causes, as autoimmune skin diseases or allergic skin diseases, such a psoriasis, allergic contact dermatitis or atopic dermatitis, also share an increase in the oxidative stress of the skin, by the production of oxygen radicals and another oxidative species as nitrogen oxide (NO) of peroxinitrite, as a way of example.

Abnormalities associated with inflammation comprise a large, unrelated group of disorders which underlie a vast variety of human diseases. However, the objective of the present invention is not focused in preventing / treating inflammation specifically, but in a particularly selected group of indications, all of them related with the skin, which are treated by applying topical formulations, more particularly those skin diseases caused by exposure to radiation. Moreover, the state of the art has disclosed drugs administered orally that might be useful for treatment of inflammatory disorders, however, those drugs administered orally would never exert their potential therapeutic effects on a far away target tissue as the skin.

QUER has been disclosed (Rubia Casagrande, Sandra R. Georgetti, Waldiceu A. Verri Jr., Daniel J. Dorta, Antônio C. dos Santos, Maria J. V. Fonseca_[2006]; J. Photochem Photobiol B: Biology 84: 21-27 and Alena Svobodová, Jitka Psotová, Daniela Walterová [2003]; Biomed. Papers 147(2): 137-145) to be effective in the prevention of the deleterious effects of UV radiation on the skin. However, the absorption spectrum of QUER shows most of its maximum peaks at wavelengths in the range of UVA (320-400 nm), not in the range of UVB (290-320 nm), precisely wherein the absorption spectrum of PTER has its maximum area.

In the state of the art (WO01/43705 A2) a combination of some members of the chemical family of stilbene, not specifically PTER, with retinoids, is used for skin care.

Present invention gives a new step forward, toward the solution of the above problems, developing compositions focused on the prevention and/or in the treatment of skin diseases, injures or damages, based on PTER as main active compound and more particularly in combinations of PTER and QUER. Moreover, present invention can be also applied to mammal (human) skin under a formulation, particularly sunscreen, after-sun, anti-aging, anti-wrinkles, formulations with photoprotective purposes, before any skin disease, damage or injure, might come out.

## DESCRIPTION OF THE INVENTION

The present invention mainly refers to the use of PTER, or any acceptable salt thereof, optionally in combination with QUER, or any acceptable salt thereof, for the manufacture of pharmaceutical compositions for preventing and/or treating skin diseases, damages or injuries. Therapeutics methods of prevention and/or treatment of skin diseases, damages or injuries, comprise the administration of an effective amount of a composition comprising PTER as active compound, or any acceptable salt thereof, optionally in combination with QUER, or any acceptable salt thereof, to a subject in need of. The present invention also refers to the pharmaceutical compositions, per se, comprising PTER as active compound or any acceptable salt thereof, optionally in combination with QUER, preferably being in the form of topical formulations and more preferably in the form of liposomes; the invention also covers the process for preparing said liposome formulations.

QUER may be replaced by any other polyphenol, providing that the combination with PTER shows synergistic effect.

For the purpose of present invention, polyphenols are a group of chemical substances which might be found in plants, but not exclusively, characterized by the presence of more than one phenol unit or building block per molecule. Polyphenols are generally divided into hydrolyzable tannins (gallic acid esters of glucose and other sugars) and phenylpropanoids, such as lignins, flavonoids, and condensed tannins.

The action of PTER and, optionally, also its combination with QUER, would be exerted in a multiple way:

a) As UV filter, because both polyphenols have chemical structures which allow radiation absorption.

b) As oxygen free radical scavengers of Reactive Oxygen Species (ROS) produced during irradiation and/or during inflammatory processes.

c) As anti-inflammatory and immunomodulatory agents.

In a preferred embodiment the compositions of the invention comprise a combination of the two active compounds PTER and QUER, or any acceptable salt thereof. Therefore, the methods of prevention and/or treatment comprise the administration of an effective amount of a composition comprising both PTER and QUER, or any acceptable salt thereof, to a subject in need of it.

A "subject", as defined in present specification, is a mammal, e.g., a human.

Inflammatory processes, as defined in present invention comprise process of acute or chronic inflammation initiated by cells already present in all tissues, mainly resident macrophages, dendritic cells, histiocytes, Kuppfer cells and mastocytes. At the onset of an infection, burn, or other injuries, these cells undergo activation and release inflammatory mediators responsible for the clinical signs of inflammation. Vasodilatation and its resulting increased blood flow

cause the redness and increased heat. Increased permeability of the blood vessels results in an exudation (leakage) of plasma proteins and fluid into the tissue (oedema), which manifests itself as swelling. Some of the released mediators such as bradykinin increase the sensitivity to pain (hyperalgesia). The mediator molecules also alter the blood vessels to permit the migration of leukocytes, mainly neutrophils, outside of the blood vessels (extravasation) into the tissue. The neutrophils migrate along a chemotactic gradient created by the local cells to reach the site of injury. The loss of function is probably the result of a neurological reflex in response to pain.

In addition to cell-derived mediators, several cellular biochemical cascade systems consisting of preformed plasma proteins act in parallel to initiate and propagate the inflammatory response. These include the complement system activated by bacteria, and the coagulation fibrinolysis systems activated by necrosis, e.g. a burn or a trauma. The acute inflammatory response requires constant stimulation to be sustained. Inflammatory mediators have short half lives and are quickly degraded in the tissue. Hence, inflammation ceases once the stimulus has been removed.

The compositions of the invention may comprise any "acceptable salt" of the active compounds included therein. As used herein, said expression means those salts of compounds of the invention that are safe and effective for use in mammals, as pharmaceuticals, and that possess the desired biological activity. Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate salts. The "acceptable salts" of present invention are preferably prepared from a polyphenol compound having an acidic functional group, such as a carboxylic acid functional group, and an acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-hydroxy substituted lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxy lower alkyl)-amines, such as N,N-dimethyl-N-(2-hydroxyethyl)amine or tri-(2-hydroxyethyl)amine; N-methyl-D-glucamine; and amino acids such as arginine, lysine, and the like. The term "acceptable salt" also includes a hydrate of a polyphenol compound.

An excipient, as defined in present specification, is an inactive substance used as a carrier or vehicle for the active compounds (PTER and/or QUER) comprised in the composition. The compositions of the invention may comprise any excipient, preferably suitable for being included in topical compositions (i.e. dermatological acceptable excipient). The following auxiliary agents mentioned below can independent of each other be present in the composition of the invention: gelling agents, oils, waxes, thickening agents, hydrophilic or hydrophobic polymers, emulsifying agents, emollients, fatty acids, organic solvents, antioxidants, stabilizers, sequestering agents, acidifying or basifying agents, emulsifiers, emollients, surfactants, film formers, biological additives to enhance performance and/or consumer appeal such as amino acids, proteins, vanilla, aloe extract or bioflavinoids, buffering agents, chelating agents such as ethylenediaminetetra-acetic acid (EDTA) or oxalic acid, colorants, dyes, propellants, antifoaming agents, wetting agents, vitamins, emulsion stabilizers, pH adjusters, thickening agents, fragrances, preservatives, opacifying agents, water and/or alcohols. The aforementioned auxiliary agents for the composition of the invention are used in the usual amounts known by those skilled in the art.

Suitable oils for the compositions of the invention are selected from animal or vegetable or synthetic oils. Particularly preferred oils are selected from the group comprising liquid petrolatum, liquid paraffin, volatile and non-volatile silicone oils, isoparaffins, polyalphaolefins, fluorated and perfluorated oils.

Suitable stabilizers for the compositions of the invention can be of non-ionic, anionic, cationic and amphiphilic nature. Preferred stabilizers are selected from the group comprising polyethylenglycol (PEG) and derivatives thereof, tweens, tritons, spans, polygycerines, polyalkyl glycerides, alkyl sulfonates, aryl sulfonates, alkyl phosphates, derivatives of alkyl-betaine and phosphatidylglycerole.

Emulsifiers are preferably used in the compositions of present invention in amounts effective to provide uniform blending of ingredients of said compositions. Suitable emulsifiers include anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium acetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and acetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and/or ethoxylated cholesterol.

Emollients may be also used in the compositions of the invention in such amounts to prevent or relieve dryness. Suitable emollients include, without limitation hydrocarbon oils and waxes; silicone oils; triglyceride esters; acetoglyceride esters; ethoxylated glyceride; alkyl esters; alkenyl esters; fatty acids; fatty alcohols; fatty alcohol ethers; etheresters; lanolin and derivatives; polyhydric alcohols (polyols) and polyether derivatives; polyhydric alcohol (polyol) esters; wax esters; beeswax derivatives; vegetable waxes; phospholipids; sterols; and/or amides.

Surfactants can further be used too in the compositions of present invention. Suitable surfactants are for example those surfactants generally grouped as cleansing agents, emulsifying agents, foam boosters, hydrotropes, solubilizing agents, suspending agents and non-surfactants, which facilitate the dispersion of solids in liquids.

Film formers which may be preferably used in the compositions of present invention should keep the composition smooth and even and are preferably, without limitation. Suitable film formers are selected from the group comprising acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (vinyl methylether/maleic anhydride copolymer); PVP (polyvinylpyrrolidone); maleic anhydride polymer, vinylpyrrolidon/hexadecene copolymer; acryliclacrylate copolymer and the like.

pH adjusters may also be used in the compositions of present invention. These pH adjusters are preferably selected from: ammonium hydroxide, triethanolamine or citric acid.

Thickening agents used for the compositions of the invention preferably are selected from: candelilla, carnauba, and microcrystalline waxes, crosslinked acrylic-acid polymers, carbomer, methylhydroxyethylcellulose, hydroxypropylmethylcellulose or hydroxyethylcellulose and polyethylene thickeners.

Examples of preferred organic solvents for the compositions of present invention include lower aliphatic alcohols and polyols.

Suitable antioxidants for the compositions used in present invention are preferably selected from the group comprising ascorbic acid (vitamin C), sodium-L-ascorbate, calcium-L- ascorbate, ascorbyl palmitate, butylhydroxyanisole, butylhydroxytoluene, calcium- disodium-EDTA, isoascorbic acid, lecitine, lactic acid, polyphosphate, tocopherol (vitamin E), like [alpha]-tocopherol, [gamma]-tocopherol, [delta]-tocopherol, propylgallate, octylgallate, dodecylgallate, sodium-isoascorbate, citric acid, sodium citrate, potassium citrate and tin-11-chloride.

Gelling agents, which are preferably used in the compositions of the invention, can be natural or synthetic polymers. Natural polymers are preferably selected from: Agar-Agar, alginate, pectin, carbomer, carrageenan, casein, dextrine, gelatine, arabic gum, keratine, locust bean gum, xanthan gum and the like. Preferred synthetic polymers which can be used in the compositions of the invention are selected from: acylic acid polymers, polyacryl amides and alkylene oxide polymers.

Any route of administration can be used for administering the composition of the invention to the subject, being the preferred routes of administration intravenous, oral and topical. In a preferred embodiment the composition of the invention is a topical formulation that may be formulated in liquid or in semi-solid form, preferably as liquid, fluid, foam, cream, gel, paste, balsam, spray, ointment, lotion, conditioner, tonic, milk, mousse, emulsion, serum, oil, stick, shampoo, jelly, suspension, dispersion, lacquer, paint, elixir, drop or aerosol. In a particular embodiment, the active compounds of the invention were administered topically in a liposomal preparation. As used in the present invention, a "liposome" is an artificial vesicle that is composed of one or more concentric layers and is used especially to deliver substances (i.e. PTER and/or QUER) to the body cells.

Topical administration of the composition of the invention leads to a high bioavailability of the active compounds PTER and/or QUER, decreasing the amount of the compositions of the invention that produces a therapeutic response in 50% of the subjects to whom are administered, i.e. decreasing the effective dosage (ED50). In pharmacology, bioavailability is used to describe the fraction of an administered dose of unchanged drug that reaches the systemic circulation, one of the principal pharmacokinetic properties of drugs. Therefore, the bioavailability is one of the essential tools in pharmacokinetics, and it must be considered when calculating dosages for non-intravenous routes of administration.

Any skin disease, damage or injury, including skin cancer, can be prevented and/or treated with the compositions of the invention. In a preferred embodiment of the invention the skin diseases, damages or injuries are associated to inflammatory processes. Therefore the invention, in a preferred embodiment, is focused on preventing and/or treating subjects predisposed to or afflicted with an inflammatory dermatosis, comprising the administration of the composition of the invention to the affected skin area (lips, face or skin in general) and/or by any other alternative way of administration. The term "inflammatory dermatosis" includes a range of skin disorders, including, but not limited to, sebaceous gland disorders, papulosquamous dermatoses, allergic dermatoses, pruritic dermatoses, vascular dermatoses, bacterial dermatoses, viral dermatoses, mycolic skin infections, granulomatous dermatoses, parasitic skin dermatoses, exfoliative dermatitis, bullous dermatoses, pigmented dermatoses, photosensitive dermatoses, dermatoses caused by collagen diseases, and dermatoses due to internal diseases. The inflammatory dermatosis can also be associated with an autoimmune condition, in which case it is referred to herein as "autoimmune dermatosis."

In a preferred embodiment of the invention, the inflammatory dermatosis is a sebaceous gland disorder, e.g., an acneiform disorder such as acne vulgaris, acne conglombata, hidradenitis suppurativa, acne rosacea, seborrhea, seborrheic dermatitis, gram negative folliculitis, pyoderma faciale, steatocystoma multiplex, sebaceous hyperplasia, or rhinophyma. In a more preferred embodiment of the invention, the composition/formulation comprising PTER or any acceptable salt thereof, optionally in combination with QUER or any acceptable salt thereof, is used to treat acne vulgaris. As is well known, acne vulgaris is a chronic skin condition characterized by comedones and papules, and can be quite severe; in particularly severe cases, pustules, cysts, and permanent scarring may occur.

In still another preferred embodiment of present invention, the inflammatory dermatosis prevented and/or treated is a papulosquamous dermatosis such as, for example, psoriasis, Pityriasis rosea, tinea versicolor, or lichen planus. The method and formulations of the invention are particularly useful in treating psoriasis, an autoimmune inflammatory disorder that has proven difficult to treat with conventional agents.

In a further preferred embodiment of present invention, the inflammatory dermatosis treated is an autoimmune dermatosis that may be, by way of example, atopic dermatitis, mast cell disease, bullous pemphigoid, pemphigus vulgaris, necrotizing vasculitis, discoid lupus erythematosus, systemic lupus erythematosis, or dermatitis herpetiformis.

In other embodiments, examples of the various types of inflammatory dermatosis with which the method and formulation of the invention are effective are as follows:

- Allergic Dermatoses: contact dermatitis; photoallergic dermatitis; industrial dermatoses caused by exposure to a variety of compounds used by industry that are contact irritants; atopic eczema (infantile and adult); and dermatoses caused by drugs and nummular eczema.
- Pruritic Dermatoses: winter, senile, and essential pruritus; pruritus ani; eternal otitis; pruritis hiemalis; pruritis vulvae; and pruritus scrotae.
- Vascular Dermatoses: erythema multiforme; erythema nodosum; stasis dermatitis; purpuric dermatoses such as those associated with thrombocytopenic purpura, nonthrombocytopenic purpura, dysproteinemic purpura, actinic purpura, scorbutic purpura, and Henochs purpura; ecchymoses; stasis purpura; primary and secondary telangiectases.
- Bacterial Dermatoses: pyoderma such as impetigo, ecthyma, folliculitis, furuncles styes, carbuncles, sweat gland infections, erysipelas, erythrasma, infected ulcers, and infected eczematoid dermatitis; and bacterial dermatoses associated with systemic bacterial infections such as scarlet fever, granuloma inguinale, chancroid, tuberculosis, leprosy, gonorrhea, rickettsial diseases, actinomycosis, and syphilis.
- Viral Dermatoses: such as those caused by Herpes simplex virus, Kaposi's varicelliform eruption, zoster, chickenpox, smallpox, vaccinia, molluscum contagiosum, lymphogranuloma venereum, exanthematous diseases such as German measles, roseola, and erythema infectiosum.
- Mycolic Skin Infections: tinea cruris (superficial fungal infections of the skin in various body sites); athlete's foot (dermatophytosis of the feet caused to infection with trichophyton mentagrophytes); tinea unguium (onychomycosis); sporotrichosis; coccidioidomycosis; histoplasmosis; and North American blastomycosis.
- Granulomatous Dermatoses: sarcoidosis; granuloma annulare; reticulohistiocytoma; and silica-induced granulomas.
- Parasitic Skin Infections: scabies, cheyletiella dermatitis; demodicosis; pediculosis
- Pigmented Dermatoses: Chloasma (melasma) and vitiligo.
- Collagen Diseases: scleroderma and dermatomyositis.
- Dermatoses Due to Internal Diseases: pyoderma gangrenosum associated with ulcerative colitis, and ulcers due to diabetes.
- Photosensitive Dermatoses: Exogenous types such as drug-induced photodermatitis and contact dermatitis with photoallergic components; and endogenous types such as those associated with porphyrias and polymorphous light eruptions.

In a preferred embodiment the skin diseases, damages or injuries are caused by the exposure to radiation, either acute or chronic. Then the disease is selected among: skin cancer, melanoma, polymorphous light eruption, actinic keratosis, solar urticaria, xeroderma pigmentosum, photoageing, chronic actinic dermatitis or sunburn. The term "radiation" comprises any kind of radiation able to cause injuries or damages in the skin, for example the radiotherapy used for treating cancer or ultraviolet radiation (UV), preferably UVB emitted by the sun.

The term "prevention" as used in the present invention means to avoid fully or partially, the appearance of skin pathophysiological processes associated to oxidation stress, excessive exposure to radiation, particularly to UV radiation linked to body-tanning , in particular to UVB radiation attributed to sun-tanning, allergy, immune responses and inflammatory processes.

As used in the present invention, the term "photo-protection" refers to the administration of the composition of the invention before the subject is exposed to radiation, mainly UVB radiation, decreasing or mitigating the risk of suffering

damages. Therefore those term "photo-protection" are understood in the present invention as being comprised under the term "prevention".

On the other hand, the term "treatment" is linked to the administration of the composition of the invention after the subject has suffered the symptoms of skin disease, damage or injure. As a way of example, the term "treatment" is preferably applied to the administration of any of the compositions disclosed in present invention to a person which has been exposed to radiation, mainly UVB radiation, repairing the damaged parts of the skin and restoring its original function and integrity. Moreover term "treatment" is linked to the administration of the composition of the invention to a subject suffering any skin disease, damage or injury associated to inflammatory processes.

PTER, optionally with QUER, or any acceptable salt thereof, may be formulated in combination with other active compounds. Among those other active compounds analgesics, anti-inflammatory agents, anti-allergics, immunomodulators, healing agents and radiation filters are preferred. For the list of UV filters suitable for being ingredients of the pharmaceutical formulations of present invention accompanying to PTER and, optionally, to QUER, or any acceptable salts thereof, we refer to the UV absorbing substances (A and/or B) disclosed in WO 2008067928. Most preferred UV filters are selected from:

**Table 1**

| UV-filter | Other names |
|---|---|
| p-Aminobenzoic acid | PABA |
| Padimate O | OD-PABA, octyldimethyl-PABA, σ-PABA |
| Phenylbenzimidazole sulfonic acid | Ensulizole, Eusolex 232, PBSA, Parsol HS |
| Cinoxate | 2-Ethoxyethyl p-methoxycinnamate |
| Dioxybenzone | Benzophenone-8 |
| Oxybenzone | Benzophenone-3, Eusolex 4360, Escalol 567 |
| Homosalate | Homo methyl salicylate, HMS |
| Menthvl anthranilate | Meradimate |
| Octocrylene | Eusolex OCR, 2-cyano-3,3diphenyl acrylic acid, 2-ethylhexylester |
| Octyl methoxycinnamate | Octinoxate, EMC, OMC, Ethylmethoxycinnamate, Escalol 557, 2-ethylhexyl-paramethoxycinnamate, Parsol MCX |
| Octyl salicylate | Octisalate, 2-Ethylhexyl salicylate, Escalol 587, |
| Sulisobenzone | 2-Hydroxy-4-Methoxybenzophenone-5-sulfonic acid, 3-benzoyl-4-hydroxy-6-methoxybenzenesulfonic acid, Benzophenone-4, Escalol 577 |
| Trolamine salicylate | Triethanolamine salicylate |
| Avobenzone | 1-(4-methoxyphenyl)-3-(4-tert-butylphenyl)propane-1,3-dione, Butyl methoxy dibenzoylmethane, BMDBM, Parsol 1789, Eusolex 9020 |
| Ecamsule | Mexoryl SX, Terephthalylidene Dicamphor Sulfonic Acid |
| Titanium dioxide | CI77891 |
| Zinc oxide | |
| 4-Methylbenzylidene camphor | Enzacamene, Parsol 5000, Eusolex 6300, MBC |
| Tinosorb M | Bisoctrizole, Methylene Bis-Benzotriazolyl Tetramethylbutylphenol, MBBT |
| Tinosorb S | Bis-ethylhexyloxyphenol methoxyphenol triazine, Bemotrizinol, BEMT, anisotriazine |
| Neo Heliopan AP | Bisdisulizole Disodium, Disodium phenyl dibenzimidazole tetrasulfonate, bisimidazylate, DPDT |
| Mexorvl XL | Drometrizole Trisiloxane |
| Benzophenone-9 | Uvinul DS 49, CAS 3121-60-6, Sodium<br>Dihydroxy Dimethoxy<br>Disulfobenzophenone |

(continued)

| UV-filter | Other names |
|---|---|
| Uvinul T 150 | Octyl triazone, ethylhexyl triazone, ET |
| Uvinul A Plus | Diethylamino Hydroxybenzoyl Hexyl Benzoate |
| Uvasorb HEB | Iscotrizino Diethylhexyl butamido triazone, DBT |
| Parsol SLX | Dimethico-diethylbenzalmalonate, Polysilicone-15 |
| Isopentenyl-4-methoxycinnamate | |

Several techniques were carried out in the present invention, such as the determination of the skinfold or epidermis thickness, both after and before the exposure to radiation, in order to elucidate the effectiveness or efficiency of the compositions of the invention in preventing and/or treating skin diseases, lesions or damages. All those techniques are well-known in the art and pertain to the common general knowledge. Moreover, both techniques are routinely used for testing the ability of different compounds to prevent or treat skin diseases, damages or injuries. The smaller the thickness or the folds of the epidermis, the more effective the treatment is. Such as it can be seen in the Examples active compound PTER, or any acceptable salt thereof, can be used, for the manufacture of a topical composition for preventing and/or treating skin diseases, damages or injuries. Moreover, the combination of active compounds PTER and QUER, or any acceptable salt thereof, shows a synergistic effect improving the function of each active compound taken separately in some of the assays as explained below. Therefore, the term "synergistic" when used in connection with the present invention means that the overall therapeutic effect of a combination of PTER and QUER, or any acceptable salt thereof, when administered as combination therapy for preventing and/or treating skin diseases, damages or injuries caused by the exposure to radiation, is greater than the therapeutic effects of these active compounds when each one is administered independently.

As shown in the examples, the determination of the skinfold thickness was carried out. **Figure 1** shows that the combination of PTER + QUER, and also PTER separately, are more efficient as photoprotector, when applied before the exposure to UVB, than the commercial creams: Vichy (VICHY Capital Soleil 50+ UVB+UVA), Heliocare (Heliocare ultra 90 UVB/UVA) and Isdin (ISDIN Extrem 40 high protection UVA and UVB), the combination of PTER + QUER showing slightly better results. Those 3 commercial creams are used for comparison effects all along the present application specification. Also, along present application, RESV is also compared, in the same conditions assay, with PTER and PTER+QUER. RESV has been previously disclosed as chemoprotective agent of skin cancer (Moammir Hasan Aziz, Shannon Reagan-Shaw, Jianquiang Wu, B. Jack Longley and Nihal Ahmad; FASEB J. express article 10.1096 (2005): 1-18). In **Figure 1**, PTER alone or the combination PTER+QUER show more efficacy for skin photoprotection either after or before UVB exposure than RESV.

Therefore either the combination of PTER + QUER, or PTER separately, could be used efficiently in the prevention of skin diseases induced by exposure to sun radiation. PTER particularly absorbing the UVB component of that radiation but also QUER (see Figure 13) which, additionally, also has an absorption spectrum in the wavelength corresponding to UVA component of sun radiation, partially responsible too of carcinogenesis due to radiation (sun) exposure. Hence, the synergistic effect observed in some of the assays of present specification of the combination PTER+QUER.

Moreover, **Figure 1** also shows that the combination of PTER + QUER is more efficient in the treatment of skin diseases, injuries or damages caused by radiation, when applied after the exposure to UVB, than the commercial creams (Vichy, Heliocare and Isdin), and also than the PTER alone. Therefore the combination of PTER + QUER could be used efficiently in the treatment of skin diseases, injuries or damages induced by ultraviolet B radiation.

As shown in the examples, the measurement of the thickness of the epidermis before irradiation (24 hours) was carried out. **Figure 2** shows that the use of PTER separately or in combination with QUER is more efficient as photoprotector, when applied before the exposure to UVB, than the commercial creams (Vichy, Heliocare and Isdin). Therefore PTER separately or in combination with QUER could be used efficiently in the prevention of skin diseases induced by ultraviolet B radiation.

Moreover, **Figure 2** also shows that the combination of PTER + QUER, or PTER separately, are more efficient in the treatment of skin diseases, injuries or damages caused by radiation, when applied after the exposure to UVB, than the commercial creams (Vichy, Heliocare and Isdin), the combination of PTER + QUER showing slightly better results. Therefore the combination of PTER + QUER, as well as PTER separately, could be used efficiently in the treatment of skin diseases, damages or injuries induced by ultraviolet B radiation.

As shown in the examples, the measurement of the thickness of the epidermis before irradiation (1 week) was carried out. **Figure 3** shows that the use of PTER separately is more efficient as photoprotector, when applied before the exposure to UVB, than the commercial creams (Vichy, Heliocare and Isdin). Therefore PTER separately could be

used efficiently in the prevention of skin diseases, damages or injuries induced by ultraviolet B radiation.

Moreover, **Figure 3** also shows that the combination of PTER + QUER, and PTER separately, are more efficient in the treatment of skin diseases, injuries or damages caused by radiation, when applied after the exposure to UVB, than the commercial creams (Vichy, Heliocare and Isdin), the combination of PTER + QUER showing the best results. Therefore the combination of PTER + QUER, as well as PTER separately could be used efficiently in the treatment of skin diseases, injuries or damages induced by ultraviolet B radiation.

Therefore, the first embodiment of the present invention refers to PTER, optionally in combination with QUER, or any acceptable salt thereof, for use in the prevention and / or treatment of skin diseases, damages or injuries. In a preferred embodiment the present invention also refers to PTER, optionally in combination with QUER, or any acceptable salt thereof, for use in the prevention and / or treatment of skin cancer. Another preferred embodiment of the invention refers to PTER in combination with QUER, or any acceptable salt thereof, for use in the prevention and / or treatment of skin diseases, damages or injuries. Another preferred embodiment of the invention refers to PTER in combination with QUER, or any acceptable salt thereof, for use in the prevention and / or treatment of skin cancer. Another preferred embodiment of the invention refers to PTER in combination with QUER, or any acceptable salt thereof, for use in the treatment of skin diseases caused by the exposure to radiation. Another preferred embodiment of the invention refers to PTER in combination with QUER, or any acceptable salt thereof for use in the prevention and / or treatment of dermatosis or psoriasis. The above use is preferred by topical administration.

The second embodiment of the present invention refers to the use of PTER, optionally in combination with QUER, or any acceptable salt thereof, for the manufacture of a pharmaceutical composition for the prevention and/or treatment of skin diseases, damages or injuries. In a preferred embodiment the present invention refers to the use of PTER, optionally in combination with QUER, or any acceptable salt thereof, for the manufacture of a pharmaceutical composition for the prevention and/or treatment of skin cancer. In a preferred embodiment the present invention refers to the use of PTER in combination with QUER, or any acceptable salt thereof for the manufacture of a pharmaceutical composition for the prevention and / or treatment of skin diseases, damages or injuries. In a preferred embodiment the present invention refers to the use of PTER in combination with QUER, or any acceptable salt thereof, for the manufacture of a pharmaceutical composition for the prevention and / or treatment of skin cancer. In a preferred embodiment the present invention refers to the use of PTER in combination with QUER, or any acceptable salt thereof, for the manufacture of a pharmaceutical composition for the treatment of skin diseases caused by the exposure to radiation. In a preferred embodiment the present invention refers to the use of PTER in combination with QUER, or any acceptable salt thereof, for the manufacture of a pharmaceutical composition for the prevention and / or treatment of dermatosis or psoriasis. Preferred manufactured pharmaceutical compositions are the ones to be administered topically.

The third embodiment of the present invention refers to a pharmaceutical composition comprising PTER, for topical administration and, more preferably, formulated in the form of liposomes. In a preferred embodiment the pharmaceutical composition comprises PTER formulated in combination with QUER. In a preferred embodiment the pharmaceutical composition is formulated in the form of sunscreen or after-sun formulations.

The fourth embodiment of the present invention refers to a process for manufacturing liposome formulations that comprises: dissolving the active compound PTER, or any acceptable salt thereof, and lecithin in an organic solvent, evaporating the solvent, adding PBS buffer, collecting and sonicating the liposomes formed, diluting 1:1 with an emulsion stabilizer, preferably a carbomer and more preferably Carbopol, optionally adding a preservative, preferably Phenonip. In a preferred embodiment the process for manufacturing liposome formulations also comprises the dissolution of QUER, or any acceptable salt thereof, in combination with PTER in the fist step. Carbopol is a carbomer. Carbomer is also a generic name for synthetic polymers of acrylic acid used as emulsion stabilizers or thickening agents in pharmaceuticals and cosmetic products. They may be homopolymers of acrylic acid, crosslinked with an allyl ether pentaerythritol, allyl ether of sucrose, or allyl ether of propylene.

Phenonip is a preservative clear liquid that works in a multitude of applications. It's unique in that it's soluble in oil and dispersible in water. This preservative is especially effective when used in conjunction with oil based products but also works extremely well in aqueous solutions. This means that it will also work in products, like balms and salves that contain only oils. Phenonip is also able to withstand a broader temperature range. Tests show that it can be heated to sterilization temperatures without degrading the quality of its preservative effects. It works in a wide ph range of 3 - 8. The usage rate varies from1% to 3% of weight of total formula (depending on the application). Phenonip is composed of and should be labeled as: phenoxyethanol, methylparaben, ethylparaben, butylparaben, propylparaben.

The fifth embodiment of the present invention refers to a method for the prevention and/or treatment of skin diseases, damages or injuries that comprises the administration of PTER, optionally in combination with QUER, or any acceptable salt thereof. In a preferred embodiment the present invention refers to a method for the prevention and/or treatment of skin cancer that comprises the administration of PTER, optionally in combination with QUER, or any acceptable salt thereof. In a preferred embodiment the present invention refers to a method for the prevention and / or treatment of skin diseases, damages or injuries that comprises the administration of PTER in combination with QUER, or any acceptable salt thereof. In a preferred embodiment the present invention refers to a method for the prevention

and / or treatment of skin cancer that comprises the administration of PTER in combination with QUER, or any acceptable salt thereof. In a preferred embodiment the present invention refers to a method for the treatment of skin diseases caused by the exposure to radiation that comprises the administration of PTER in combination with QUER, or any acceptable salt thereof. In a preferred embodiment the present invention refers to a method for the prevention and / or treatment of dermatosis or psoriasis that comprises the administration of PTER in combination with QUER, or any acceptable salt thereof. More preferably, the method of the invention is applied topically.

## BRIEF DESCRIPTION OF THE FIGURES

**Figure 1.** It shows the skinfold thickness (%) of mice treated with different compositions before (left column) or after (right column) being exposed to UVB. Commercial creams (Vichy, Heliocare -Helio- and Isdin). C+UVB= Control irradiated without any treatment; C Lipo+UVB= Control irradiated wherein only liposomes (without containing any active compound) applied on. RESV= Resveratrol; P+Q= PTER+QUER.

**Figure 2.** It shows the thickness ($\mu$m) of the epidermis treated with different compositions before (left column) or after (right column) being exposed to irradiation (24 hours). Commercial creams (Vichy, Heliocare -Helio- and Isdin). Control= No irradiation, no treatment; C+UVB= Control irradiated without any treatment; C Lipo+UVB= Control irradiated wherein only liposomes (without containing any active compound) applied on. RESV= Resveratrol; P+Q= PTER+QUER.

**Figure 3.** It shows the thickness ($\mu$m) of the epidermis treated with different compositions before (left column) or after (right column) being exposed to irradiation (1 week). Commercial creams (Vichy, Heliocare -Helio- and Isdin). C Lipo+UVB= Control irradiated wherein only liposomes (without containing any active compound) applied on. RESV= Resveratrol; P+Q= PTER+QLTER.
In all Figures 1-3 the asterisk * shown in the bars histogram represents a difference statistically significant ($P < 0.05$), when compared with control values.

**Figure 4.** It shows the increase in the skinfold with respect to day 0 (1 week after pre-exposure to TNCB, and the start of continues exposure on alternate days) in the trial regarding the induction of experimental atopic dermatitis with TNCB.

**Figure 5.** It shows the loss of transepidermal fluid 24 hours after the 4° exposure to TNCB, in the trial regarding the induction of experimental atopic dermatitis with TNCB.

**Figure 6.** It shows the lesions 24 hours after the 4° exposure to TNCB, in the trial regarding the induction of experimental atopic dermatitis with TNCB.

**Figure 7.** It illustrates the protocol developed in the trial regarding the induction of experimental atopic dermatitis with TNCB.

**Figure 8.** It shows the increase of skinfold in reference to day 0 of TPA treatment, in the trial regarding the induction of experimental psoriasis with TPA.

**Figure 9.** It shows the loss of transepidermal fluid (TEWL) 24 hours after the 7° exposure to TPA, in the trial concerning the induction of experimental psoriasis with TPA.

**Figure 10.** It shows the evolution of the lesions on the exposure to TPA, in the trial concerning the induction of experimental psoriasis with TPA.

**A.** Image of a mouse 24 hours after 3 daily exposures of 2 nmoles of TPA;

**B.** Image of the same mouse 24 hours after 6 daily exposures of 2 nmoles of TPA;

**C.** Image of real psoriasis on a patient's finger (Image from the Dermatology Image Atlas);

**D.** Images of real psoriasis on a patient's knee (Image from the Dermatology Image Atlas).

**Figure 11.** It shows the protocol developed in the trial concerning the induction of experimental psoriasis with TPA.

**Figure 12.** It shows the protocol developed in the trial concerning the induction of experimental hypersensitivity by contact to oxazolone.

**Figure 13.** It shows the absorption of polyphenols, specifically the absorption spectrum of different 1mM solutions of each of the polyphenols (PTER, Resveratrol and QUER), in pure ethanol, in the trial regarding the assessment of the mechanical filter capacity of polyphenols.

**Figure 14.** It shows the condition of the mice after chronic exposure to UV-B in the trial regarding the assessment of the photoprotector role of the polyphenols. It is a photograph of the different experimental groups after 25 weeks of chronic exposure to 180 mJ/cm$^2$ (three times per week). Both the reference compounds and the ones subject of this study (10μmoles/mouse of polyphenols, or 200μl of each photoprotector) were applied to the back of the mice for 20 minutes prior to their exposure to UV-B.

**Figure 15.** It shows the evolution of the development of lesions in the trial regarding the assessment of the photoprotector role of the polyphenols. First signs of precancerous lesions of more than 1 mm of diameter in the different experimental groups after 30 weeks of chronic exposure to 180 mJ/cm$^2$ (three times per week). Both the reference compounds and the ones subject of this study (10μmoles/mouse of polyphenols, or 200μl of each photoprotector) were applied to the back of the mice for 20 minutes prior to their exposure to UV-B.

**Figure 16.** It shows the anatomopathological study of the skin after chronic exposure to UV-B. Anatomopathological evaluation of histological sections of mice after 30 weeks of chronic exposure to 180 mJ/cm$^2$ (three times per week). Both the reference compounds and the ones subject of this study (10μmoles/mouse of polyphenols, or 200μl of each photoprotector) were applied to the back of the mice for 20 minutes prior to their exposure to UV-B.

**Figure 17.** It shows the skinfold after the continue administration of treatments, in the trial regarding the assessment of the effect of the different polyphenols on dermatitis. The application of the treatments 10 μmoles/mouse of polyphenols and 0.5 μmoles/mouse of dexamethasone was performed daily on a third of the back of the mice for 6 consecutive days. The skinfold was measured with the help of a vernier caliper.* $p>0.001$ with regards to the control. ANOVA followed by a Tukey test.

**Figure 18.** It shows the histological sections representing the different experimental conditions in the trial regarding the assessment of the protection from severe exposure to radiation UV-B. The application of treatments (10μmoles/mouse of polyphenols, or 200μl of each photoprotector) was performed 20 minutes before or after irradiation with 360mJ/cm$^2$ of UV-B. The mice were sacrificed 24 hours after irradiation to UV-B. This figure indicates the changes in the thickness of the epidermis.

**Figure 19.** It shows the increase in the skinfold 24 hours after the first administration of TNCB in the trial regarding the protection in an experimental model of atopic dermatitis induced by TNCB. The application of treatments (10μmoles/mice of polyphenols, 0.5 μmoles/mice of dexamethasone) was performed 24 hours before (day 0) to the application of 100 μl of 1% TNCB on mice already sensitized to 1 TNCB. The skinfold was measured with a vernier caliper and the increase with respect to day 0 was calculated. * $p>0.05$ with respect to the control; $^+p>0.05$ with respect to the control treated with TNCB. ANOVA followed by a Tukey test.

**Figure 20.** It shows the evaluation of the lesions in the model of atopic dermatitis in the trial regarding the macroscopic assessment of the lesions. The application of treatments (10μmoles/mice of polyphenols, 0.5 μmoles/mice of dexamethasone) was performed 24 hours before the application of 100 μl of 1% TNCB on mice already sensitized to 1 TNCB. The measure of the loss of water for epidemic transmission (TEWL) was evaluated with the Tewameter system TM 210 (CK electronics, Germany). * $p>0.05$ with respect to the control; $^+p>0.05$ with respect to the control treated with empty Liposomes. ANOVA followed by a Tukey test.

**Figure 21.** It shows the evaluation of the mice in the model of atopic dermatitis in the trial regarding the macroscopic assessment of the lesions. The application of treatments (10μmoles/mice of polyphenols, 0.5 μmoles/mice of dexamethasone) was performed 24 hours before the application of 100 μl of 1% TNCB every 2 days on mice already sensitized to 1 TNCB, and up to 4 applications of TNCB.

**Figure 22.** It shows the increase of skinfold 24 hours of administration of Oxazolone, in the trial regarding the

protection against a model of contact hypersensivity induced by Oxazolone. The application of treatments (1 μmoles/ mouse of polyphenols, 20 mg of diprosone) was performed 24 hours prior (day 0) of the application of 20 μL of 2.5% oxazolone in ethanol on the mice's ears, previously sensitized to Oxazolone. The skinfold was measured with a vernier caliper and the increase with respect to day 0 was calculated. * p>0.05 with respect to the control treated with Oxazolone; +p>0.05 with respect to the control treated with diprosone before the application of Oxazolone. ANOVA followed by a Tukey test.

**Figure 23.** It shows the evolution of the increase of the skinfold in reference to day 0 throughout the procedure, in the trial regarding the protection against a model of psoriasis induced by TPA. The application of the treatments (10μmoles/mice of polyphenols, 0.5 μmoles/mice of dexamethasone) was performed 30 minutes prior the application of 2 nmol/mouse of TPA. This administration guideline was repeated over 6 consecutive days. The skinfold was measured with a vernier caliper and the increase with respect to day 0 was calculated.

**Figure 24.** It shows the increase in the skinfold 24 hours after the first administration of TPA in the trial regarding the protection against a model of psoriasis induced by TPA. The application of the treatments (10μmoles/mice of polyphenols, 0.5 μmoles/mice of dexamethasone) was performed 30 minutes prior the application of 2 nmol/mouse of TPA. The skinfold was measured with a vernier caliper and the increase with respect to day 0 was calculated. * p>0.05 with regards to the control. ANOVA followed by a Tukey test.

**Figure 25.** It shows the skinfold 24 hours after the third administration of TPA in the trial regarding the protection against a model of psoriasis induced by TPA. The application of the treatments (10μmoles/mice of polyphenols, 0.5 μmoles/mice of dexamethasone) was performed 30 minutes prior the application of 2 nmol/mouse of TPA. The skinfold was measured with a vernier caliper and the increase with respect to day 0 was calculated. * p>0.05 with respect to the control; $^+$p>0.05 with respect to the group TNCB. ANOVA followed by a Tukey test.

**Figure 26.** It shows the macroscopic view of the mice in the psoriasis model in the trial regarding the protection against a model of psoriasis induced by TPA. The photos were taken **(A)** 24 hours after the administration of TPA and **(B)** 24 hours after the 7ª TPA administration. The application of the treatments (10μmoles/mice of polyphenols, 0.5 μmoles/mice of dexamethasone) was performed 30 minutes prior the application of 2 nmol/mouse of TPA.

**Figure 27.** It shows the evaluation of descaling 24 hours after seven administrations of TPA in the trial regarding the protection against a model of psoriasis induced by TPA. The application of the treatments (10μmoles/mice of polyphenols, 0.5 μmoles/mice of dexamethasone) was performed 30 minutes prior the application of 2 nmol/mouse of TPA. The descaling aspect of the skin was quantified in a scale of 0-3, being 0, nothing; 1, mild; 2, moderate; 3, severe. And the medium of each group indicated the degree of inflammation.

**Figure 28.** It shows the histological analysis of the psoriasis procedure. The application of the treatments (10μmoles/ mice of polyphenols, 0.5 μmoles/mice of dexamethasone) was performed 30 minutes prior the application of 2 nmol/ mouse of TPA. Image of a microscopic view of a cut of 5 μm with staining of hematoxylin-eosin from mice at 24 hours (A.) and 72 hours (B.) after being treated with 2 nmoles of TPA daily over 7 days.

## DETAILED DESCRIPTION OF THE INVENTION

Studies for evaluating the capacity of the compositions of the invention of preventing skin diseases (such as skin cancer) induced by ultraviolet B radiation were carried out by acute irradiation at a dose of 360 mJ/cm$^2$.

### Protocol for exposure to UVB radiation.

To make the exposure to UVB a specially upgraded camera (Crosslinker equipped with 5 tubes which emit 80% of the radiation as UVB, with a maximum emission at 312 nm) was used.

UVB Acute exposure protocols were used: Mice underwent a single exposure of 360 mJ/cm2. The study was conducted at 24 hours after exposure. This protocol was used to evaluate the photoprotective effect of the compounds studied. With the same acute-phase protocol mice from all groups were irradiated. Subsequently mice remained in the animal storage area for a week to see the evolution of the inflammatory response and edema.

### Administration and dosage of the compounds under study.

For the UVB exposure studies (chronic or acute) a topical administration to the back of the mouse was conducted.

The commercial sunscreens were applied directly on the mouse. The polyphenols in the study were administered in a liposomal preparation diluted 1:1 with Carbopol polymer which will be the vehicle for these liposomes. Each polyphenol dose administered was 10 μmol/mouse, as free acid.

**Parameters indicators of skin damage.**

1. Edema formation: skinfold thickness was determined with a caliper 24 hours after the first exposure.

2. Study of the thickness of the epidermis after exposure to acute irradiation (360mj/$cm^2$ 24h hours and 7 days after irradiation).

3. Histological studies: measuring, in the pictures obtained from histological sections, the thickness of the epidermis.

**Histological studies.**

For studies of acute exposure skin samples were taken from the back of the mouse. These samples were fixed with 4% paraformaldehyde (24 hours) and embedded in paraffin. Following cutting the samples were stained with hematoxylin and eosin.

**Study of the absorption spectrum of the different compounds.**

A solution of 1mM of each polyphenol was prepared (PTER, Resveratrol and QUER) in absolute ethanol (HPLS quality; Quima) and the absorption spectrum was studied between 200 and 800 nm using a Multiskan Spectrum (ThermoScientific).

**Procedures of photo carcinogenesis (chronic exposure to radiation UV-B).**

The role of UV-B radiation in tumor formation is well known. The continuous exposure to UV-B promotes the formation of oxidative / nitrosative stress, along with alterations in DNA, proteins, etc. that activates various transcription factors (among them NF-κB and AP-1), increase in the telomerase activity, stress, etc. which in turn induces carcinogenesis.

Furthermore, the exposure to UV-B increases the expression and levels of antiapoptotic proteins, which correlate with the increase in proliferation and certain inflammation markers.

The mice chosen for this study were SKH-1. These mice have a greater risk of developing tumors following chronic exposure to low doses of UV radiation (comparable to the exposure that a human being could have on a daily basis).

The protocol used was the standardized protocol, whereby several mice were irradiated three times per week with a dose of 180 mJ/$cm^2$ with exposures for 24 weeks; however, during the trial the protocol was modified by lengthening the time of exposure to up to 28 weeks, in order to obtain more evidence of the photoprotector effect on the different treatments used.

The dose of exposure was 180 mJ/cm2, since it had been determined in previous trials that this was the minimal erythemic dose (MED) for this type of rodent.

A range of different commercial photoprotectors were included in this study:

- Vichy: Capital Soleil SPF-50+ (Vichy Laboratoires).
- Isdin: Photoprotector Isdin Extrem (SPF-40 (Isdin SA)
- Heliocare: Heliocare gel Protection Ultra 90 (Difa Cooper S.P.A.)

Both the reference compounds and the ones subject of this study were applied to the back of the mice for 20 minutes prior to their exposure to UV-B.

The groups, of 20 mice, for this assessment were:

- Not treated.

- Reference compound Vichy prior: 200 μl of Isdin applied 20 minutes prior to irradiation.

- Reference compound Isdin prior: 200 μl of Isdin applied 20 minutes prior to irradiation.
- Reference compound Heliocare prior: 200 μl of Heliocare applied 20 minutes prior to irradiation.
- Medium prior: 200 μl of vacuumed Liposomes applied 20 minutes prior to irradiation.
- Resveratrol prior: 200μL of Resveratrol Liposomes (10 μmol in total) applied 20 minutes prior to irradiation.
- PTER prior: 200μL of PTER Liposomes (10 μmol in total) applied 20 minutes prior to irradiation.
- QUER prior: 200μL of QUER Liposomes (10 μmol in total) applied 20 minutes prior to irradiation.
- PTER + QLTER prior: 200μL of PTER + QUER Liposomes (10 μmol in total) applied 20 minutes prior to irradiation.

UV-B induces edema and erythema in the short-term. This inflammation is critical for the hyperproliferation process.

Skin cells exposed to UV have higher levels of the nuclear transcription NF-κB factor, this factor control the output of COX-2 and iNOS, among others, a reason why UV-B is a clear cause of skin inflammation.

With the purpose of assessing the effects of the photoprotective compounds studied, female mice SKH-1 of between 6-8 weeks old were subjected to a single exposure of 360 mJ/cm.

For the ultraviolet B (UVB) irradiation exposure, a camera specifically prepared, available at our laboratory was used (Crosslinker; Bio-Link BLX 254) equipped with 5 tubes that produce 80% of the UVB radiation, with a maximum emission of 312 nm.

The two protocols of UV-B exposure used were:

- Intense exposure after a single exposure of 360 mJ/cm$^2$. The study was carried out 24 hours after the exposure. This protocol was used to assess the photoprotective effects of the compounds studied.
- Intense exposure after a single exposure of 360 mJ/cm$^2$. The study was carried out 1 week after the exposure. The mice were kept in the shed for a week to observe the evolution of the inflammatory and edematous response.

As reference, several commercial photoprotective products were used (Vichy, Isdin and Heliocare). The reference compounds as well as the products object of this study, were applied on the back of the mice for 20 minutes after or 20 minutes before the irradiation with UV-B, to determine if the reaction observed was due to an effect of the solar filter or not.

The groups, of 5 mice, for this assessment were:

- Not treated.
- Reference compound Vichy prior: 200 μl of Isdin applied 20 minutes prior to irradiation.
- Reference compound Vichy after: 200 μl of Isdin applied 20 minutes after irradiation.
- Reference compound Isdin prior: 200 μl of Isdin applied 20 minutes prior to irradiation.
- Reference compound Isdin after: 200 μl of Isdin applied 20 minutes after irradiation.
- Reference compound Heliocare prior: 200 μl of Heliocare applied 20 minutes prior to irradiation.
- Reference compound Heliocare after: 200 μl of Heliocare applied 20 minutes after irradiation.
- Medium prior: 200 μl of vacuumed Liposomes applied 20 minutes prior to irradiation.
- Medium after: 200 μl of vacuumed Liposomes applied 20 minutes after irradiation.
- Resveratrol prior: 200μL of Resveratrol Liposomes (10 μmol in total) applied 20 minutes prior to irradiation.
- Resveratrol after: 200μL of Resveratrol Liposomes (10 μmol in total) applied 20 minutes after irradiation.
- PTER prior: 200μL of PTER Liposomes (10 μmol in total) applied 20 minutes prior to irradiation.
- PTER after: 200μL of PTER Liposomes (10 μmol in total) applied 20 minutes after irradiation.
- QUER prior: 200μL of QUER Liposomes (10 μmol in total) applied 20 minutes prior to irradiation.
- QUER after: 200μL of QUER Liposomes (10 μmol in total) applied 20 minutes after irradiation.
- PTER + QLTER prior: 200μL of PTER + QUER Liposomes (10 μmol in total) applied 20 minutes prior to irradiation.
- PTER + QUER after: 200μL of PTER + QUER Liposomes (10 μmol in total) applied 20 minutes after irradiation.

At 24 hours or 1 week after the administration of the different treatments the mice were sacrificed and their skin fixed with 4% PFA.

**Ear thickness.**

The edema was calculated as the difference between the thickness that was produced in the ears, before the outcomes of the reaction, and their thickness 24 hours later, and it was measured with a vernier caliper.
The change was expressed as the relative increase in relation to day 0.

**Macroscopic assessment of the skin.**

The mice were observed daily to detect any changes on the appearance of the skin or any general changes.
Also, on certain specific days prior to sacrificing them, the mice were assessed by a veterinarian who analyzed the skin lesions, qualifying them in function of:

- Erythema: redness of the skin conditioned by inflammation due to the excess of blood irrigation by the dilation of blood vessels.
- Excoriation: or irritation of the skin with flaky aspect, increase in the descaling of the skin which could result in hyperkeratosis.
- The depth of the lesion that could reach the dermis producing soars or ulcers.

Each variable was evaluated on a scale of 0-4, 0, for none; 1, for mild; 2, for moderate; 3, marked; and 4, very marked. The addition of the 3 values of each mouse indicated the degree of inflammation.
During the photocarcinogenesis process, the presence of different tumors was followed and quantified throughout the process. Any lesion of more than 1mm of diameter was considered a lesion.

**Microscopic analysis.**

Histochemical techniques were used to study the skin tissues of the different procedures, at a microscopic level.
The tissues were fixed in 4% formaldehyde in PBS over 24 h at 22°C. After the tissues were fixed, they had to be dehydrated in large amounts of ethanol and placed in a hydrophobic and xylene mixture to allow the paraffin to penetrate the tissues to provide sufficient consistency for slicing them thinly in a microtome.
The samples in paraffin were processed with the microtome obtaining slices of about 5-10 μm. The samples were spread over a drop of gelatin at 37°C and were collected in gelatinized slides.
For other procedures it was necessary to eliminate the paraffin over temperatures of 60° C and by washing the samples with xylene, and by rehydrating them with ethanol.
Hematoxylin is a cationic colorant while eosin is an anionic colorant belonging to the xanthens. Therefore the nucleus is stained in blue and the cytoplasm in pink.
The protocol used for staining the samples was:

1. Incubation with Mayer's Hematoxylin for 10-15 minutes.
2. Wash under running water until bluish.
3. Incubate with Eosin (1%) for 2-4 minutes.
4. Eliminate the remaining eosin in ethanol 70°
5. Dehydration.
6. Rinse with xylol for 3 minutes.
7. Let the trays dry out on the stove at 37°.

**Statistical analysis.**

The results were expressed with the means ± standard deviation. The standard deviation was represented in the form of error bars, in some cases only positive semi bars were shown to simplify the graphics.
The data was analyzed based on the variations (ANOVA) of one or more factors or tests as appropriate. The homogeneity of the variants was analyzed with the Levene test. The null hypothesis was accepted for all the values of the tests in which value F was not significant with a value of p greater than 0.05. The data, for which the value F was significant, was analyzed with the Turkey test with a p value of 0.05.
The computer programs used were Excel for the calculations and graphics and for the statistics SPSS version 14.0 for Windows (SPSS Inc) was used, the University of Valencia has a user's license for this.

## EXAMPLES

### Example 1. Experimental animal model.

Strain SKH-1 mice provided by Charles River Company were used as experimental animals. They are hairless immunocompetent mice. The experimental groups were formed with female mice aged 3 to 4 weeks as showed in **Table 2.** Each experimental group of photoprotection consisted of between 5 and 6 animals. In these studies, each experimental group consisted of 20 mice.

**Table 2**

| GROUP | NAME | TREATMENT | UVB EXPOSURE |
|---|---|---|---|
| I | Negative control | Liposomal vehicle | No |
| IIt | UVBa | Liposomal vehicle | 20 min after treatm. |
| IIa | UVBb | Liposomal vehicle | 20 min before treatm. |
| IIIt | RESa | RES liposomes | 20 min after treatm. |
| IIIa | RESb | RES liposomes | 20 min before treatm. |
| IVt | PTERa | PTER liposomes | 20 min after treatm. |
| IVa | PTERb | PTER liposomes | 20 min before treatm. |
| Vt | QUERa | QUER liposomes | 20 min after treatm. |
| Va | QUERb | QUER liposomes | 20 min before treatm. |
| VIt | (PTER+QUER)a | PTER+QUER liposomes | 20 min after treatm. |
| VIa | (PTER+QUER)b | PTER+QUER liposomes | 20 min before treatm. |
| VIIt | ISDINa | | 20 min after treatm. |
| VIIa | ISDINb | | 20 min before treatm. |
| VIIIt | HELICAREa | | 20 min after treatm. |
| VIIIa | HELIOCAREb | | 20 min before treatm. |
| IXt | VICHYa | | 20 min after treatm. |
| IXa | VICHYb | | 20 min before treatm. |
| b refers to "before"<br>a refers to "after" | | | |

Moreover, *in vivo* trials were performed on 4 week-old naked female mice SKH1-E from Charles River, or on 6-8 week BALB/c mice.

The studies took place at the University of Valencia, Central Support Service of Experimental Research (CSSER) in the area of animal experimentation from the animal production service, excepting the studies performed by TNO Quality of Life (Leiden, Netherlands), of hypersensitivity to oxazoline.

Once the animals were acclimatized after a period of 2 weeks, the experiments were performed in rooms equipped with protective barriers, under controlled conditions, with temperatures of: 22 $\pm$2 °C, relative humidity: 65-80%, 12 changes of air/hour and a photoperiod of 12/12 hours, in compliance with the current international regulation [European Community Council Directive (86/609, OJ L 358. 1, December 12, 1987), National Health Institute (Guide for the Care and Use of Laboratory Animals, NIH Publ. No. 85-23, 1985, USA)].

The animals were sacrificed by cervical dislocation. Samples were taken from the hindquarters for histology, and were fixed in 4% formaldehyde overnight. Parts of the samples were kept in liquid $N_2$ for the intense UV-B exposure procedures, and for subsequent evaluation for inflammation and oxidative/nitrosative stress.

Subsequently, 20 $\mu$l of blood were taken and added to 80 $\mu$l of N-ethylmaleimide (NEM).

100 $\mu$l perchloric acid (PCA) 4% were added after incubation for about 10 min. Samples were preserved in ice after vigorous shacking. Finally all samples were centrifuged at 13000 rpm for 15 minutes at 4°C. Supernatants were collected and stored at -20 °C until analysis of the GSH (reduced form of glutathione) / GSSG (oxidized form of glutathione) ratio. The GSH/GSSG ratio is a parameter that reflects the intracelular redox status, and the intracellular thiol redox

staus in particular. Therefore changes in this ratio are used to evaluate oxidative stress.

Four samples were taken from skin of the hindquarters. One for histology (was fixed overnight in 4% formaldehyde) and 3 were frozen with liquid nitrogen for biochemical analysis.

**Example 2. Preparation of liposomes.**

Regardless of the polyphenol(s) the methodology of preparation of liposomes were equally used: soy lecithin; dichloromethane as organic solvent in the case of PTER and/or QUER, and ethyl ether as organic solvent for resveratrol due to its inability to dissolve in dichloromethane. The evaporation of organic solvent was performed using a rotary evaporator connected to vacuum and a temperature of 40 °C. After removal of organic solvent, liposomes were resuspended in PBS diluted to 1 mM. In the final liposome concentration, before adding the Carbopol, the concentration of each polyphenol was of 20 μM polyphenols as determined by HPLC / MS-MS.

The steps for preparing the liposomes can be summarized as follows:

1. Weight polyphenols and lecithin

| PTER | QUER | RESV | LECITHIN |
|---|---|---|---|
| 0,512gr | 0,676gr | 0,458gr | 2,376 |

2. Dissolve the polyphenol and lecithin in 40ml of dichloromethane (in the case of PTER and/or QUER), or 140 ml of ethyl ether (in the case of resveratrol).
3. Introduce the solution into a rotary evaporator flask and attached to the device. Once all the solvent evaporated cut the expected vacuum and wait for 15 min. to eliminate all traces of solvent which may have become.
4. Add 20ml of diluted PBS (1mM) in the rotary evaporator flask and re-attach the device but this time without vacuum.
5. The liposomes formed are collected in a 50 ml tube.
6. Sonication of liposomes (6 seconds, 25 cycles, with an interval of 3 seconds between each cycle; *high intensity*).
7. Dilution 1:1 with Carbopol (final concentration of 10 μmoles/200 μl)
8. To avoid possible contamination is added Phenonip (6ml of Phenonip per 1000 ml of preparation).

Control liposomes were made in the same manner as those of PTER and QUER, but only contain lecithin (2.376g).

The procedures to obtain liposomes were performed in our laboratories in cooperation with the Colloids Research Unit (Dept. of Chemistry and Physics; Univ. Valencia).

The liposomes can be loaded with a variety of active principles, such as polyphenols. Due to their ability to penetrate the skin, they can carry these active principles for long distances within the skin, and stop at a determined spot. This is the reason why liposomes can be used in dermatological therapy. There is a variety of products that contain polyphenols that come from green tea (Replenix; Kaplan cosmetic Surgery Centers), and resveratrol that comes from grapes (Resveraderm; SesDERMA Laboratorios).

A mixture of soy lecithin was used to obtain liposomes; this is a natural mixture of about 90% phosphatidylcholine and other fatty acids. The soy lecithin (99% pure) was provided by GUINAMA S.L (Valencia, Spain).

Polar organic solvents were used for solubility, such as dichloromethane (Sigma) for the PTER (CML Europe BV) and QUER (Sigma), and ether (Sigma) for the Resveratrol (Sigma).

The ideal mixture to obtain liposomes contained:

- 5.9 (w/v) of lecithin, 1.28 (w/v) of PTER and 1.69 (w/v) of QUER, dissolved in dichloromethane.
- 1.69 (w/v) of lecithin and 1.15 (w/v) of Resveratrol dissolved in ether.

The microscopic packaging of the different polyphenols was achieved by the dehydration of the soy lecithin at 40° C in a vacuum rotary evaporator, which was later hydrated with a solution of 1 mM phosphate pH 7 at 20°C for a final concentration of 0.1 mM per compound in the final solution.

The liposomes were standardized in relation to the size of the particles by observing the morphology via microscope. The concentration of the polyphenols in the liposomes suspension was quantified by HPLC-MS/MS.

The solution was stabilized Carbopol 940 (Guinama) to a dilution 1:1, adding 0.6% (v/v) of antimicrobial preservative Phenonip (Guinama), with which was obtained a solution of liposomes with 10 μmoles for each polyphenol/200 μl.

The administration of the liposomes was done by applying and smoothing a homogeneous film of the cream to the back of the mouse with an automatic micropipette.

The compounds were applied in accordance with the administration protocol, described in each procedure,

which were:

- Empty liposomes: liposomes without charge.
- PTER Liposomes: 1 μmol/ 20μL of cream.
- QUER Liposomes: 1 μmol/ 20μL of cream.
- PTER + QUER Liposomes: 1 μmol of each compound/ 20μL of cream.
- Resveratrol Liposomes: 1 μmol/ 20μL of cream.

**Example 3. Determination of skinfold 24 h after irradiation.**

The skinfold was measured by making a fold on the skin from the back of the mice, from the axillae to the hips; the fold was measured with a vernier caliper.

The increase in the fold was calculated by the difference between the initial point, before the effects of the reaction, and the thickness in each point.

**Fig. 1** shows the results obtained in measuring the thickness of the skin of the hindquarters mice (where the various treatments have been applied). The results were statistically analyzed by ANOVA and contrasted by the Tukey test and grouped according to the significant differences. Standard deviations are incorporated in **Fig. 1.** As shown in **Figure 1,** the combination of PTER + QUER and also PTER separately, are more efficient as photoprotector, when applied before the exposure to UVB, than the commercial creams (Vichy, Heliocare and Isdin) and that RESV. Therefore either the combination of PTER + QUER or PTER separately could be used efficiently in the prevention of skin diseases induced by ultraviolet B radiation.

Moreover, **Figure 1** also shows that the combination of PTER + QUER or PTER separately are more efficient for the treatment of skin diseases, damages or injuries due to radiation, when applied after the exposure to UVB, than the commercial creams (Vichy, Heliocare and Isdin) and that RESV. Therefore the combination of PTER + QUER could be used efficiently in the treatment of skin diseases, damages or injuries induced by ultraviolet B radiation.

**Example 4. Measuring the thickness of the epidermis after irradiation (24 hours).**

To measure the thickness of the epidermis to evaluate possible hyperplasia, a computer program was used, Image J, calibrated with the assistance of a Neubauer camera which allowed the measuring of the epidermis. Results obtained were statistically analyzed by ANOVA and contrasted by the Tukey test and grouped according to the significant differences. As shown in **Figure 2,** the use of PTER alone is more efficient as photoprotector, when applied before the exposure to UVB, than the commercial creams (Vichy, Heliocare and Isdin). Therefore PTER alone could be used efficiently in the prevention of skin diseases, injuries or damages induced by ultraviolet B radiation.

Moreover, **Figure 2** also shows that the combination of PTER + QUER is also more efficient for the treatment of skin diseases, damages or injuries caused by radiation, when applied after the exposure to UVB, than the commercial creams (Vichy, Heliocare and Isdin) and that RESV. Therefore the combination of PTER + QUER, as well as PTER separately, could be used efficiently in the treatment of skin diseases, damages or injuries induced by ultraviolet B radiation. Control in this experiment means no radiation and no treatment.

**Example 5. Measuring the thickness of the epidermis after irradiation (1 week).**

The evaluation of skin thickness was carried out by using computer software (IMAGE J). Results obtained by ANOVA were contrasted by the Tukey test and grouped according to the significant differences. As shown in Figure 3, the use of PTER separately is more efficient as photoprotector, when applied before the exposure to UVB, than the commercial creams (Vichy, Heliocare and Isdin). Therefore PTER separately could be used efficiently in the prevention of skin diseases, damages or injuries induced by ultraviolet B radiation.

Moreover, **Figure 3** also shows that the combination of PTER + QUER is more efficient too, for the treatment of skin diseases, damages or injuries caused by radiation, when applied after the exposure to UVB, than the commercial creams (Vichy, Heliocare and Isdin) and that RESV. Therefore the combination of PTER + QUER, as well as PTER separately could be used efficiently in the treatment of skin diseases induced by ultraviolet B radiation.

**Example 6. Induction of experimental atopic dermatitis with TNCB.**

As to agent "2-chloro-1,3,5-trinitrobenzene or 2,4,6-trinitrochlorobenzene" (TNCB), also commonly known as Pycril chloride, this is a chemical agent that induces an allergic process combined with the inflammation of the skin of the mice. Furthermore, the continuous administration of TNCB ointment increased the production of T-lymphocytes, mastocytes and IgE in the serum, as it occurs among human patients with atopic dermatitis.

To produce atopic dermatitis lesions, the protocol described by Matsumoto was used, applying 100μL of a solution of 1% TNCB in acetone [3 applications of 33 μL (with the aid of a dispenser of an automatic micropipette) to prevent the volume from spreading and to maintain the lesion area more localized] on the back of the SKH-1 mice.

In contrast to the protocol proposed by Matsumoto, where the treatment with TNCB lasted 32 days following a prior testing period with TNCB, a protocol consisting of one test with TNCB and a week later 4 doses followed by TNCB every 2 days was developed. Shorter protocols produced an increase in the skinfold as shown in Figure 4, as in the loss of transepidermal fluid as shown in Figure 5, these lesions being obvious at macroscopic level as shown in Figure 6.

Therefore, the protocol developed in this procedure was the one shown in Figure 7. As a standard treatment, the following was used Dexamethasone (from Sigma Solution 25mM in acetone). The administration of the products to be tested and the standard treatment were given in alternate days, beginning 24 hours prior to the first continuous application of TNCB and after the mice were exposed to TNCB (1 week prior).

The groups, of 5 mice, for this assessment were:

- Not treated.
- Reference compound: 0.5 μmoles of Dexamethasone (20 μl of one solution 25mM in acetone).
- Medium: 200 μl of vacuumed Liposomes.
- Resveratrol: 200μL of Resveratrol Liposomes (10 μmol in total).
- PTER: 200μL of PTER Liposomes (10 μmol in total).
- QUER: 200μL of QUER Liposomes (10 μmol in total).
- PTER + QUER: 200μL of PTER Liposomes + QUER (10 μmol in total).

At 24 hours (day 9) after the last administration of the different treatments the mice were sacrificed and their skin fixed with 4% PFA.

**Example 7. Experimental induction of psoriasis with TPA.**

For the induction of psoriasis, the agent "12-O-tetradecanoylphorbol-13 acetate" (TPA) was used. In relation to the agent "12-O-tetradecanoylphorbol-13 acetate" (TPA), also commonly known as "phorbol 12-myristate 13-acetate" (PMA), this is the diester of phorbol and a strong promoter of tumors usually used in biomedical research to activate the transduction of signals via the kinase enzyme of protein C (PKC).

In reference to the dose of TPA needed to develop the disease on the hairless mice SKH-1, some authors have used elevated doses of 10-17 nmoles/mouse, the achieved reproduction of hyperplasia is similar to how it occurs in Balb/C mice, previously shaved, with psoriasis, and this model is widely used in the studies of psoriasis. However, these models were used to evaluate biochemical or molecular parameters after only one administration of TPA. Since our objective was to assess the progress of the lesions caused by psoriasis in combination with the use of polyphenols, a model of continuous exposure and a weaker concentration of TPA were used, on a daily dose of 2 nmoles of TPA over a period of a week, which allowed us to observe the progress of the psoriasis.

The induction was performed with 2 nmol TPA /mouse on an area of 1 $cm^2$, applying 10μL of the 200μM solution in acetone. This application protocol was carried out taking into account that when applying 20 μL as stated in the model, the volume increased considerably and it lost its concentration, therefore it was decided to make it more concentrated.

Shorter protocols produced an increase in the skinfold as shown in **Figure 8,** as in the loss of transepidermal fluid (TEWL) as shown in **Figure 9,** these lesions being obvious at macroscopic level as shown in **Figure 10.**

Therefore, the protocol developed in this procedure was the one shown in **Figure 11.** As a standard treatment, Dexamethasone was used, (from Sigma Solution 25mM in acetone). The administration of the standard treatment and the products to be tested were given 30 minutes prior to the application of TPA, both beginning on the same day.

The groups, of 6 mice, for this assessment were:

- Not treated.
- Reference compound: 0.5 nmoles of Dexamethasone (20 μl of one solution 25mM in acetone).
- Medium: 200 μl of vacuumed Liposomes.
- Resveratrol: 200μL of Resveratrol Liposomes (10 μmol in total).
- PTER: 200μL of PTER Liposomes (10 μmol in total).
- QUER: 200μL of QUER Liposomes (10 μmol in total).
- PTER + QUER:: 200*L of PTER Liposomes + QUER (10 μmol in total).

At 24 hours (day 7) after the last administration of the different treatments and TPA, 3 mice from each group were sacrificed and their skin fixed with 4% PFA. The remaining mice were kept under the treatment with no exposure to TPA, to verify if the skin recovered faster on the treatment than on its own. At 24 hours (day 9) after the last administration

of the different treatments the rest of the mice were sacrificed and their skin fixed with 4% PFA.

**Example 8. Experimental induction of hypersensitivity by contact to Oxazolone.**

The application of oxazolone after sensitization to the same substance produces a reaction of hypersensitivity by contact, which appears as an eczematous reaction, with infiltration of lymphocytes and macrophages, with edema of the epidermis.

The BALB/c mice are sensitized on day 0 through the application, on the shaved abdomen, of 100 μL of oxazolone at 1.5% in acetone (w/v). Prior to the induction for hypersensitization, the thickness of the ear was measured with the help of a vernier caliper (Mitutoyo micrometer). The reaction was caused on day 7 by applying 20 μL of 2.5% oxazolone in ethanol (10 μL to each side of the right ear).

Therefore, the protocol developed in this procedure was the one shown in Figure 12. As a control measure the left ear was used, to which 20 μL of ethanol was applied. And a standard treatment, Diprosone® cream was used: (Schering Plough B.V., Utrecht, The Netherlands), that contains 0.5 mg de betamethasone/ g.

The groups, of 8 mice, for this assessment were:

- Not treated.
- Reference compound: 10 mg of Diprosone® cream to each side of the ear, applied 30 minutes after applying oxazolone on day 7.
- Medium: 10 μl of empty Liposomes to each side of the ear applied 30 minutes prior to the application of oxazolone on day 7.
- Resveratrol prior: 10μl of Resveratrol Liposomes to each side of the ear (1 μmol in total) applied 30 minutes prior to the application of oxazolone on day 7.
- Resveratrol after: 10μl of Resveratrol Liposomes to each side of the ear (1 μmol in total) applied 3-4 hours after the application of oxazolone on day 7.
- PTER prior: 10μl of PTER Liposomes to each side of the ear (1 μmol in total) applied 30 minutes prior to the application of oxazolone on day 7.
- PTER after: 10μl of PTER Liposomes to each side of the ear (1 μmol in total) applied 3-4 hours after the application of oxazolone on day 7.
- QUER prior: 10μl of QUER Liposome to each side of the ear (1 μmol in total) applied 30 minutes prior to the application of oxazolone on day 7.
- QUER after: 10μl of QUER Liposomes to each side of the ear (1 μmol in total) applied 3-4 hours after the application of oxazolone on day 7.
- PTER + QUER prior: 10μl of PTER + QUER Liposomes to each side of the ear (1 μmol in total) applied 30 minutes prior to the application of oxazolone on day 7.
- PTER + QUER after: 10μl of PTER + QUER Liposomes to each side of the ear (1 μmol in total) applied 3-4 hours after the application of oxazolone on day 7.

24 hours after the administration of the oxazolone (day 1), the mice were sacrificed and both ears were fixed with 4% PFA.

**Example 9. Transepidermal water loss.**

Some skin conditions are characterized by compromising the mechanical properties of the skin, such as in scleroderma and psoriasis.

Nonetheless, the objective evaluation of these characteristics is complex, and the measuring of the loss of transepidermal water (PTEA or TEWL) assessed through the Tewameter TM 210 system (CK electronics, Germany) is considered an indicator of the functions of cutaneous "barriers" (g/h/cm2).

The physical foundation of this type of measurement is based in the law of diffusion established by Adolf Fick in 1885:

$$\frac{dm}{dt} = -D \times A \times \frac{dp}{dx}$$

whereas:

A = surface in $m^2$

m = transportation of water (in g)
t = time (h)
D = constant of diffusion (=0.0877 g/m·h·mm Hg)
p = pressure of atmospheric vapor (mm Hg)
x = distance from the skin to the measuring point (m)

The law of diffusion dm/dt indicates the mass by $cm^2$ transported in an interval of time, which is proportional to area A and the variation of concentration by the distance dc/dx. D represents the coefficient of diffusion of water vapor in the air. This law is valid only within a zone of homogeneous diffusion, which has the approximate shape of an empty cylinder. The resulting gradient density is registered indirectly by two sets of detectors (temperature and relative humidity) analyzed by a microprocessor.

In the probe of the Tewameter TM 210 system, the temperature and hydration detectors, as well as the circuitry for measuring and data for calibration, are located in the interior of the sensor. The head of the sensor is made by a hollow and narrow cylinder, (diameter = 10 mm, length = 20 mm) and its function is to reduce the air turbulence in the sensor.

After immobilizing the mice, the probe was place on their back, in the same area treated with different chemical agents. TEWL readings were performed continuously until the metrics were stabilized, which can produce between 30-60 seconds of readings.

**Example 10. Photoprotector and anticarcinogenic effect during chronic exposure to UV-B irradiation.**

**Mechanical filter capacity of polyphenols.**

With the purpose of studying the capacity of the polyphenols used in this study, we decided to evaluate the absorption of light throughout the ultraviolet light spectrum as shown in **Figure 13.**

It was confirmed that Resveratrol as well as PTER have peaks or maximum absorption in the range of Ultraviolet B, in contrast QUER is absorbed in larger proportion in the wavelengths within the UV-A.

**Photoprotector role of polyphenols.**

The following phase of the study was to evaluate the photoprotector component (adding the polyphenols before exposure to UV-B) against the possible repairing capability of the polyphenols against photocarcinogenesis (adding polyphenols after exposure to UV-B).

For this purpose, the photoprotector capacity was evaluated after chronic exposure to UV-B as shown in **Figure 14.** The mice were subject to 3 exposures per week over 28 weeks to a dose of 180 $mJ/cm^2$ of UV-B per exposure. With this protocol neoplasias are induced which begin to show starting on the twelfth week. Throughout the procedure the precancerous lesions were evaluated and quantified as shown in the **Figure 15.**

If we compare the effects of the different treatments in relation to the signs of lesions, we will be able to appreciate than the control group as well as the one treated with empty liposomes, beginning on week 12 of continuous exposure to UV-B, over 50% of the mice had tumors. In contrast, the treatment with polyphenols delayed the showing of tumors until approximately the 20th week in the case of Resv and QUER. However, under Pter and after 30 weeks of chronic exposure to UV-B, only 10% of the mice presented lesions, with Pter alone or in combination with Quer. This protective effect was superior to the one found in the commercial photoprotectors.

In relation to the number of lesions per mouse, Pter and the one combined with Quer were the ones that provided the most protection, since the number of lesions per mouse were null in comparison to any of the other treatments. The Table 3 below shows the lesions per mouse: first signs of precancerous lesions of more than 1 mm of diameter in the different experimental groups after 30 weeks of chronic exposure to 180 mJ/cm2 (three times per week). Both the reference compounds and the ones subject of this study (10$\mu$moles/mouse of polyphenols, or 200$\mu$l of each photoprotector) were applied to the back of the mice for 20 minutes prior to their exposure to UV-B. Group of 20 mice.

Table 3

| | n° of tumor/mouse x group(mean) | | |
|---|---|---|---|
| weeks | 20 | 24 | 30 |
| Control | 4,4 | 9,2 | 13,8 |
| Free liposomes | 5,7 | 8,2 | 12,9 |
| PTER liposomes | 0,0 | 0,1 | 0,0 |
| QUER liposomes | 0,3 | 2,2 | 4,6 |
| Resveratrol liposomes | 0,3 | 1,2 | 3,8 |
| Pter + Quer liposomes | 0,0 | 0,1 | 0,1 |
| Vichy: | 0,0 | 0,3 | 0,7 |
| Heliocare: | 0,0 | 0,5 | 1,06 |
| Isdin: | 0,1 | 0,5 | 0,6 |

**Anatomopathological studies.**

In relation to the histopathological exam of the tumors induced by chronic irradiation to UV in the SKH1 mice, it was confirmed that the treatment with UV-B produced a strong acanthosis due primarily to the inflammatory process; this gave way to the first signs of tumors, mostly of carcinoma type and not melanoma.

The great majority of the two non-melanoma skin cancers are: basal cell carcinoma and flaky cell carcinoma.

The use of commercial photoprotectors protected mainly from inflammation, however only Vichy diminished the possible development of tumors.

The use of polyphenols had anti-inflammatory and anti-tumor formation effects; PTER was the compound with greatest benefits. However, when compared with its counterpart resveratrol, a great difference was observed at the level of anti-inflammatory and anti-tumor formation. It also presented better results when compared to different commercial photoprotectors.

About the combination of Pter and Quer, no differences were found between the uses of Pter alone or combined in relation to its photoprotector effect, but there was a difference in relation to its anti-inflammatory effect as it will be discussed later. As it is represented in **Figure 16,** the **Table 4** below shows the anatomopathological study of the skin after chronic exposure to UV-B, specifically after 30 weeks of chronic exposure to 180 mJ/cm2 (three times per week). Both the reference compounds and the ones subject of this study (10$\mu$moles/mouse of polyphenols, or 200$\mu$l of each photoprotector) were applied to the back of the mice for 20 minutes prior to their exposure to UV-B. Medium of groups of 20 mice. Every parameter was measured according to the degree of seriousness (+) light-(+++) serious.

**Table 4**

| | | UV | PQ + UV | Resv + UV | Isdin + UV | Heliocare + UV |
|---|---|---|---|---|---|---|
| **Tumor** | **(no/mouse x group)** | 13.8 | 0.1 | 3.8 | 0.7 | 1.1 |
| **Stroma** | **Dysplasia** | | + | ++ | ++ | ++ |
| | **Fibrosis** | Reactive | | | | |
| | **Acanthosis / Acantholysis** | + / +++ | | +++ / ++ | ++ | +++ / ++ |
| | **Parakeratosis** | +++ | | + | | + |
| | **Inflammation** | +++ | | ++ | ++ | ++ |

**Example 11. Effect on dermatitis.**

Prior to evaluating the results of different treatments on different models of dermatitis, the possible side effects of continues administration of polyphenols on the skin of the mice had to be evaluated as shown in **Figure 17.** For this purpose, the dose for this daily evaluation was administered over 6 consecutive days.

As it can be observed, the treatment alone did not affect the skinfolds after a chronic treatment, however, the chronic use of corticoids (such as dexamethasone) did have side effects on the structure of the skin.

**Protection from acute exposure to radiation UV-B.**

Having seen the anti-inflammatory characteristic of the polyphenols as protection against photocarcinogenesis, the anti inflammatory role was studied after severe exposure to UV-B. The results obtained with measures of the thickness of the skin of the four mice's backs are shown in **Figure 1.**

The polyphenols Pter and Resv individually and the combination with Pter+Quer, showed a beneficial effect after the exposure to UVB irradiation since the formation of the edema was reduced.

This increase in the thickness of the skin could be due to the volume caused primarily by some inflammatory process, or by the hyperproliferation of the cells of the epidermis. Therefore it was needed to measure the thickness of the epidermis as shown in **Figure 2.**

A significant difference in the thickness of the epidermis of the mice treated with polyphenols was noticed with respect to the group treated only with UV-B. In fact, the treatment with PTER with or without QUER produced better results than even commercial photoprotectors and Resveratrol.

In relation to the time the treatment was applied, there were no important differences between applications before or after exposure to UV-B, except for Resveratrol, which showed a different result in relation to the time of application with regards to its good photoprotection role but not as a repairer.

Also, when the thickness of the epidermis was analyzed a week after irradiation of 360 $mJ/cm^2$ of UV-B, it was noticed that the mice treated with polyphenols before UV-B exposure were protected from the damage of UV-B, they maintained the same results of the thickness of the epidermis obtained 24 hours after irradiation, there were no statistical differences. If the polyphenols were applied after irradiation the same results were obtained, except for the QUER that gave a worst prognosis if applied after the exposure to UV-B, the results were similar to the results observed in commercial photoprotectors as shown in **Figure 3 and Figure 18.**

**Protection in an experimental model of atopic dermatitis induced by TNCB.**

The increase in the skinfolds 24 hours after the first administration of TNCB was studied as shown in **Figure 19.**

In this model it was impossible to measure the skinfold, since there were lesions produced throughout the process that did not allow the correct measurement of the thickness of the skin. However, it was noticed that there were increases in the skinfold 24 hours after the first exposure to TNCB, 1 week after the previous sensitization with TNCB. As it can be observed in the figure, the dexamethasone as well as the PTER (alone or combines with QUER) decreased their inflammatory effect due to the TNCB agent.

It should also be observed that the empty liposomes had no effect on the skinfolds, showing that the liposomes on their own do not work as mechanical filters, disallowing the TNCB to reach their target producing the expected lesions.

**Macroscopic assessment of the lesions.**

The lesions were evaluated with the purpose of quantifying the differences between the different treatments, as shown in **Figure 20** and **Figure 21.**

It was observed that from the beginning of the first continuous application of TNCB, there were changes in the aspect of the skin, there were changes in the level of hydration from the start of the first exposure to TNCB and throughout the process.

In short term, the analysis of the TEWL values showed that for the treatment with TNCBA, as well as for the pre-treatment with dexamethasone or empty liposomes there was a significant statistical increase of the hydration of the epidermis in relation to the basal values.

However, what was also showed by the analysis of the results was that in the case of treatments with polyphenols, especially in the combination Pter-Quer, there were no significant statistical differences with relation to untreated skin (basal value). This shows that the polyphenols have a hydrating effect on the epidermis, more so than water alone, or a protective effect towards aggression produced by TNCB.

At this time, the differences stopped being statistically significant with relation to the values obtained with TNCB, possibly because there were lesions in all the groups, including the group treated with dexamethasone

In relation to the lesions, there is indication that the low levels of degree of the lesion was because in contrast with other models, the protocol for administering the TNCB was of a lower dose, which also produced minor lesions but more similar to real lesions.

The most important fact to remark on will be in regards to the possible prevention or cure of atopic dermatitis, the dexamethasone did not produce any beneficial effect in relation to the index of lesions or in relation to the increase of the skinfold. Te PTER produced less serious lesions than with the use of dexamethasone or with respect to the lack of treatment.

**Protection against a model of contact hypersensitivity induced by Oxazolone.**

Skin disorders such as contact hypersensitivity, atopic dermatitis and psoriasis, are characterized by hyperproliferative and inflammatory disorders of the skin.

The current treatment for skin disorders includes the use of anti-inflammatory agents such as glucocorticoids and antiproliferative agents. In this study it is stated that the use of natural polyphenols produces a clinical improvement of dermatitis as shown in

**Figure 22.**

The effects of PTER on skin disorders was established according to procedures performed on animals that showed a similar hypersensitivity to contact on the inflammation of the dermis, measured by use of oxazolane in the prevention of the edema of the ear.

The results of this pharmacological trial *in vivo* that emulated skin immune-related inflammatory disorders indicates that the polyphenols in particular PTER, applied on the lesion (applied after the administration of oxazolane) could prevent inflammatory changes as a result of inflammation induced by oxazolone. Similar results can be observed with diprosone.

**Example 12. Effect on psoriasis induced by TPA.**

**Study of edema in psoriasis**

Since the application of TPA on the skin produces vasodilatation, infiltration of leukocytes and edema, it is interesting to measure not just the variation of the skinfold as the weight of the tissue but as a perimeter of the cutaneous inflammation, as shown in **Figure 23.**

While evaluating the increase of skinfold throughout the procedure (6 continues days) 3 different groups were observed. The first group corresponds to the Control group and the one treated with dexamethasone (0.5 μmoles/mouse 30 minutes prior to administering TPA), there were no differences among these through the treatment, indicating that the dexamethasone prevented the development of psoriasis. The second group would be the one formed by TPA, empty liposomes and the Resv, where at least throughout the first half of the procedure the evolution was similar with an increase in the formation of edema.

And lastly, the third group formed by the group of Pter, Quer and Pter+Quer, where an important decrease in the increase of skinfolds was noticed, under the TPA group throughout, and significantly different at least after 24 hours of the first administration as shown in **Figure 24**.

As it can be observed by the increase of the skinfold, the application of TPA on the mice' skin produces edema, a significant strong inflammation, 24 hours after the administration. The application of the product in reference, the dexamethasone (0.5 μmoles/mouse) 30 minutes prior to the topical administration of TPA, reduced significantly the increase of the skinfold.

At the same time, treatments with PTER, QUER and the combination of both also significantly diminished the appearance of edema. It is important to note that the use of empty liposomes (not charged) had no effect upon the appearance of edema, indicating that the effect observed by the Pter, Quer or Pter + Quer, was not due to the composition of the liposomes but instead it was due to a specific effect of the polyphenols. This effect was corroborated in comparisons with liposomes charged with the polyphenol in question, resveratrol; this did not produce any effect on the increase of the skinfold in comparison to the TPA group.

If the skinfold is compared after 3 administrations of TPA, where the lesions at a macroscopic level are obvious, it is obtained the **Figure 25**.

At this time, it was obvious how the use of Pter, Quer and a combination of both produce a diminishing effect on the inflammation produced by TPA.

**Macroscopic assessment of the lesions.**

The lesions were evaluated with the purpose of quantifying the differences between the different treatments as shown in **Figure 26**.

A numeric value was given to the lesions according to the excoriation of the lesions 24 hours after the 7 administrations of TPA, since this was the only alteration that was noticed, there were no indications of erythema or ulcers.

The flaky aspect of the skin was quantified in a scale of 0-3, being 0, nothing; 1, mild; 2, moderate; 3, severe. And the medium of each group indicated the degree of inflammation as shown in **Figure 2**.

It can be observed that only the groups with PTER on its own or in combination with QUER, were the groups with fewer indexes of excoriation or descaling.

**Histological analysis of the lesions.**

This analysis is illustrated in **Figure 28**.

Histologically, there is elongation of the rete ridge with thickness of the inferior part (psoriasiform hyperplasia), elongation and edema of the papillae, suprapapillary epidermal atrophy with spongiform intracorneal pustules (of Kogoj), lack of granular stratum or hypogranulosis, parakeratosis, Munro's intraepidermal microabscess, dilated and tortuous papillary capillaries, perivascular lymphohistiocytic infiltration and few eosinophils and plasmocytes.

Psoriasis is chronic inflammatory dermatosis, characterized clinically by lesions in the form of papules and scaling erythema, with abundant pearly-white- descaling. Its course is fussy although generally it has a chronic evolution with periods of exacerbation and remission. There have been different clinical descriptions, although generally there is not a set frame since there can be variations within the same patient. We can see many of these during examination or during different evolution stages of the illness.

For years it has been known that the hyperplasia of the epidermis in psoriasis is secondary to the hyperproliferation of keratinocytes, which is characterized by the number of germinating cells in the proliferation stage and of the fragmentation of growth in the epidermis. An increase in the index of mitosis and the number of cells during the clinical phase has been detected.

The typical histopathological characteristic of a stable psoriasis lesion is the "epidermal psoriasiform hyperplasia" (Lever's Histopathology of the Skin. 10th edition). It is characterized by a regular epidermal acanthosis with rete ridges wide at the base and narrow on the upper part, where there is usually a thinning of the suprapapillary epidermis.

In our model with induction of psoriasis in the group with TPA this papillomatosis was observed; however, there was no narrowing of the suprapapillary epidermis. Also hyperkeratosis parakeratosis was observed.

When the cuts were made to the mice that were retired from the treatment, there was no evidence of clear lesions at a macroscopic level; therefore we did not analyze the level of excoriation since it could have led to errors. However, the histological analysis indicated that the skin was not healthy skin. Therefore when thoroughly analyzed it, it was confirmed that when psoriasis lesions enter a period of involution, the normal cronification tends to be recovered, the parakeratosis begins to disappear and the epidermis begins to thin out. Also, the papillomatosis disappears although the morphology did not reach the level to be that of normal skin.

At comparing the different groups of treatment, it was observed that the group treated with dexamethasone presented very thin epidermis without papillomatosis or hyperkeratosis. In some points there was also evidence of lesions

to the sebaceous glands.

In reference to the group treated with PTER, the most important difference was that there were no signs of papillomatosis, but there was acanthosis due to a massive proliferation. And if we compared with the group treated only with TPA or with empty liposomes, the acanthosis was less pronounced, same as with the parakeratosis.

This did not occur with the group treated with Resveratrol, a similar structure of PTER, since in this group the proliferation and acanthosis was less sharp than in the group treated with PTER. Besides, comparing the different treatments, the group treated with Resveratrol resembles more the group treated with TPA, than the group with PTER.

Also, the combination of PTER with QUER found a phenotype similar to the one found with the PTER alone.

## Claims

1. PTER, 3,5-dimethoxy-4'-hydroxy-trans-resueratrol optionally in combination with QUER 3,3, 4' 5,6-pentahydroxy flavona, or any acceptable salt thereof, for use in the prevention and / or treatment of skin diseases, damages or injuries by topical administration.

2. PTER, optionally in combination with QUER, or any acceptable salt thereof, according to claim 1, for use in the prevention and / or treatment of skin cancer.

3. PTER in combination with QUER, or any acceptable salt thereof, according to claim 1, for use in the prevention and / or treatment of skin diseases, damages or injuries.

4. PTER in combination with QUER, or any acceptable salt thereof, according to claim 1, for use in the prevention and / or treatment of skin cancer.

5. PTER in combination with QUER, or any acceptable salt thereof, according to claim 1, for use in the treatment of skin diseases caused by the exposure to radiation.

6. PTER in combination with QUER, or any acceptable salt thereof, according to claim 1, for use in the prevention and / or treatment of dermatosis, preferably psoriasis.

7. Pharmaceutical composition comprising PTER, optionally in combination with QUER, for topical administration.

8. Pharmaceutical composition, according to claim 7, wherein PTER is formulated in combination with QUER.

9. Pharmaceutical composition, according to claim 7, formulated in the form of sunscreen or after-sun formulations.

10. Pharmaceutical composition comprising PTER, according to claim 7, formulated in the form of liposomes.

11. Process for manufacturing liposome formulations that comprises:

    a) Dissolving the active compound PTER, or any acceptable salt thereof, and lecithin in an organic solvent.
    b) Evaporating the solvent.
    c) Adding PBS buffer.
    d) Collecting and sonicating the liposomes formed.
    e) Diluting 1:1 with an emulsion stabilizer, preferably a carbomer and more preferably Carbopol
    f) Optionally adding a preservative, preferably Phenonip

12. Process, according to claim 11, wherein QUER, or any acceptable salt thereof, is also dissolved in step (a).

## Patentansprüche

1. PTER (3,5-Dimethoxy-4'-hydroxy-trans-resveratrol), wahlweise in Kombination mit QUER (3,3,4'5,6-Pentahydroxyflavon), oder ein pharmazeutisch annehmbares Salz davon zur Verwendung bei der Prophylaxe und/oder Behandlung von Hautkrankheiten, -schäden oder - verletzungen durch topische Verabreichung.

2. PTER, wahlweise in Kombination mit QUER, oder ein pharmazeutisch annehmbares Salz davon nach Anspruch 1

zur Verwendung bei der Prophylaxe und/oder Behandlung von Hautkrebs.

3. PTER in Kombination mit QUER oder ein pharmazeutisch annehmbares Salz davon nach Anspruch 1 zur Verwendung bei der Prophylaxe und/oder Behandlung von Hautkrankheiten, -schäden oder - verletzungen.

4. PTER in Kombination mit QUER oder ein pharmazeutisch annehmbares Salz davon nach Anspruch 1 zur Verwendung bei der Prophylaxe und/oder Behandlung von Hautkrebs.

5. PTER in Kombination mit QUER oder ein pharmazeutisch annehmbares Salz davon nach Anspruch 1 zur Verwendung bei der Behandlung von durch Strahlenexposition verursachten Hautkrankheiten.

6. PTER in Kombination mit QUER oder ein pharmazeutisch annehmbares Salz davon nach Anspruch 1 zur Verwendung bei der Prophylaxe und/oder Behandlung von Dermatosen, vorzugsweise von Psoriasis.

7. Pharmazeutische Zusammensetzung, die PTER, wahlweise in Kombination mit QUER, umfasst, zur topischen Verabreichung.

8. Pharmazeutische Zusammensetzung nach Anspruch 7, worin PTER in Kombination mit QUER formuliert ist.

9. Pharmazeutische Zusammensetzung nach Anspruch 7, die in Form von Sonnenschutz- oder After-Sun-Formulierungen formuliert ist.

10. PTER umfassende pharmazeutische Zusammensetzung nach Anspruch 7, die in Form von Liposomen formuliert ist.

11. Verfahren zur Herstellung von Liposomenformulierungen, umfassend:

a) Lösen des Wirkstoffes PTER oder eines pharmazeutisch annehmbaren Salzes davon und Lecithin in einem organischen Lösungsmittel;
b) Verdampfen des Lösungsmittels;
c) Hinzufügen von PBS-Puffer;
d) Gewinnung und Beschallung der gebildeten Liposomen;
e) 1:1-Verdünnung mit einem Emulsionstabilisator, vorzugsweise einem Carbomeren und besonders bevorzugt mit Carbopol;
f) Wahlweiser Zusatz eines Konservierungsmittels, vorzugsweise Phenonip.

12. Verfahren nach Anspruch 11, wobei QUER oder ein pharmazeutisch annehmbares Salz davon auch im Schritt (a) gelöst wird.

## Revendications

1. PTER 3,5,-diméthoxy-4'-hydroxy-trans-resvératrol éventuellement combiné avec QUER 3,3,4'5,6-penta hydroxy flavone, ou n'importe lequel de ses sels acceptables, à utiliser dans la prévention et/ou le traitement de dermopathies, lésions ou blessures par administration topique.

2. PTER, éventuellement combiné avec QUER, ou n'importe lequel de ses sels acceptables, selon la revendication 1, à utiliser dans la prévention et/ou le traitement de cancer cutané.

3. PTER, combiné avec QUER, ou n'importe lequel de ses sels acceptables, selon la revendication 1, à utiliser dans la prévention et/ou le traitement de dermopathies, lésions ou blessures.

4. PTER, combiné avec QUER, ou n'importe lequel de ses sels acceptables, selon la revendication 1, à utiliser dans la prévention et/ou le traitement de cancer cutané.

5. PTER, combiné avec QUER, ou n'importe lequel de ses sels acceptables, selon la revendication 1, à utiliser dans le traitement de dermopathies provoquées par l'exposition aux radiations.

6. PTER, combiné avec QUER, ou n'importe lequel de ses sels acceptables, selon la revendication 1, à utiliser dans

la prévention et/ou le traitement de dermatoses, de préférence le psoriasis.

**7.** Composition pharmaceutique comprenant PTER, éventuellement combiné avec QUER, pour administration topique.

**8.** Composition pharmaceutique, selon la revendication 7, dans laquelle PTER est formulé en combinaison avec QUER.

**9.** Composition pharmaceutique, selon la revendication 7, formulée sous la forme d'écran solaire ou de formulations après-soleil.

**10.** Composition pharmaceutique comprenant PTER, selon la revendication 7, formulée sous la forme de liposomes.

**11.** Procédé pour fabriquer des formulations de liposomes qui consiste à :

a) Dissoudre le composé actif PTER, ou n'importe lequel de ses sels acceptables, et de la lécithine dans un solvant organique.
b) Évaporer le solvant.
c) Ajouter une solution tamponnée de phosphate.
d) Recueillir et soniquer les liposomes formés.
e) Diluer dans une proportion de 1:1 avec un stabilisateur d'émulsion, de préférence un carbomère et de préférence encore du Carbopol.
f) Éventuellement ajouter un agent conservateur, de préférence du Phenonip.

**12.** Procédé, selon la revendication 11, dans lequel QUER, ou n'importe lequel de ses sels acceptables, est également dissous dans l'étape (a).

# FIGURE 1

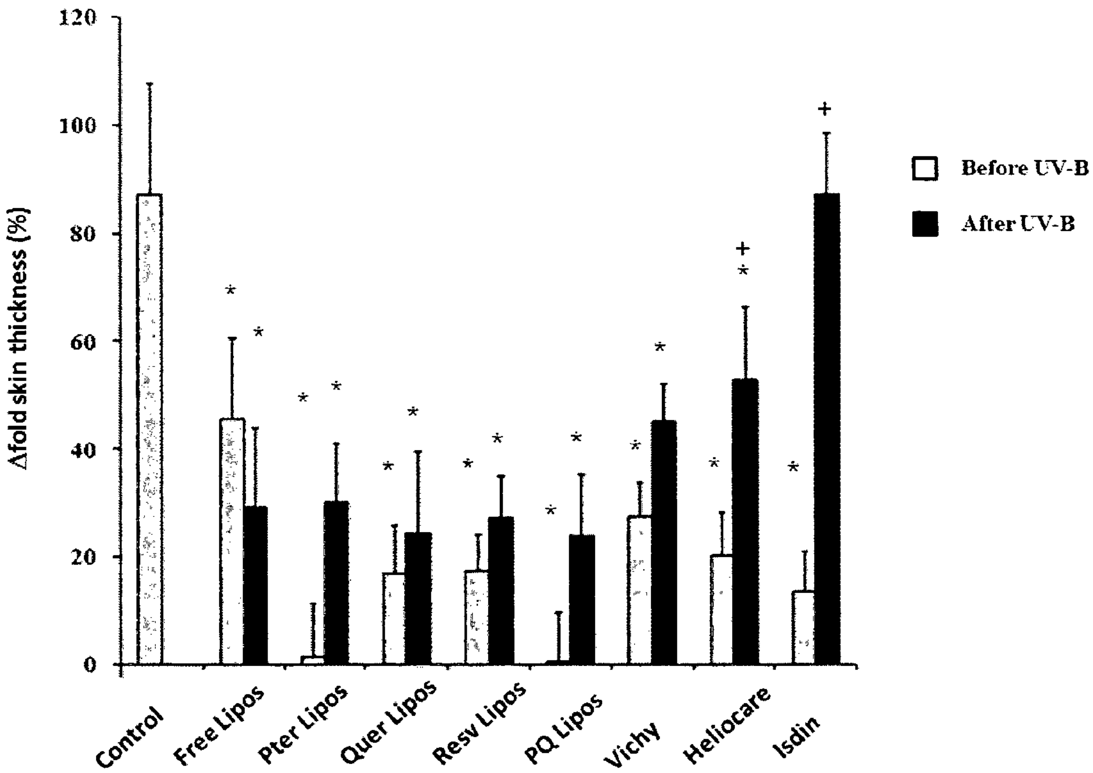
120
100
80
60
40
20
0
Δfold skin thickness (%)
Control
Free Lipos
Pter Lipos
Quer Lipos
Resv Lipos
PQ Lipos
Vichy
Heliocare
Isdin
Before UV-B
After UV-B

# FIGURE 2

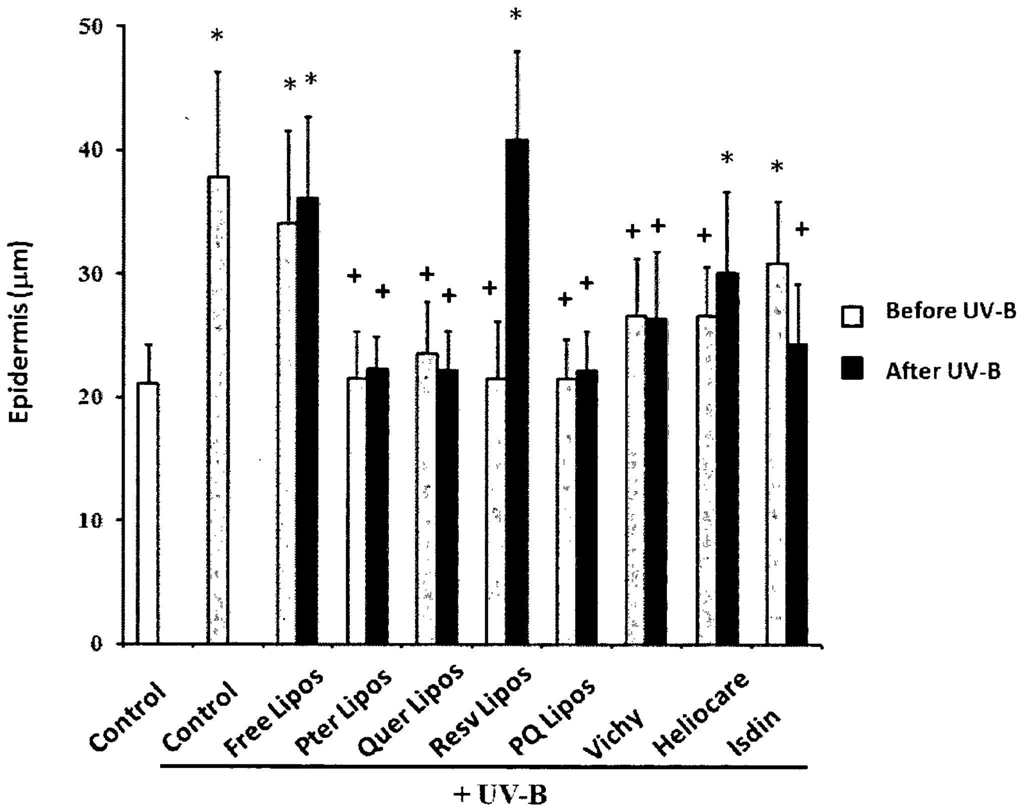
50
40
30
20
10
0
Epidermis (µm)
Control
Control
Free Lipos
Pter Lipos
Quer Lipos
Resv Lipos
PQ Lipos
Vichy
Heliocare
Isdin
+ UV-B
Before UV-B
After UV-B

# FIGURE 3

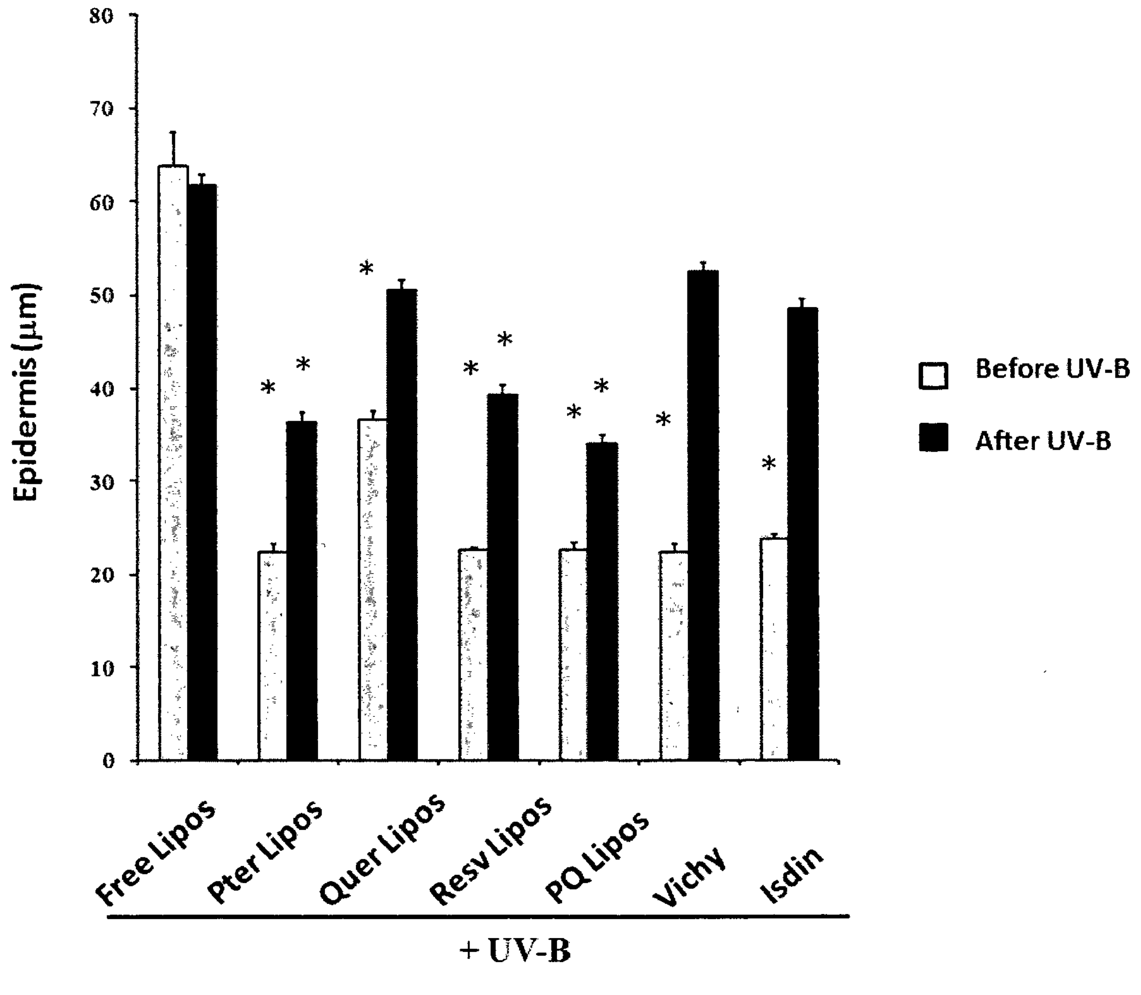
80
70
60
50
40
30
20
10
0
Epidermis (μm)
*
*
*
*
*
*
*
*
*
*
Before UV-B
After UV-B
Free Lipos
Pter Lipos
Quer Lipos
Resv Lipos
PQ Lipos
Vichy
Isdin
+ UV-B

# FIGURE 4

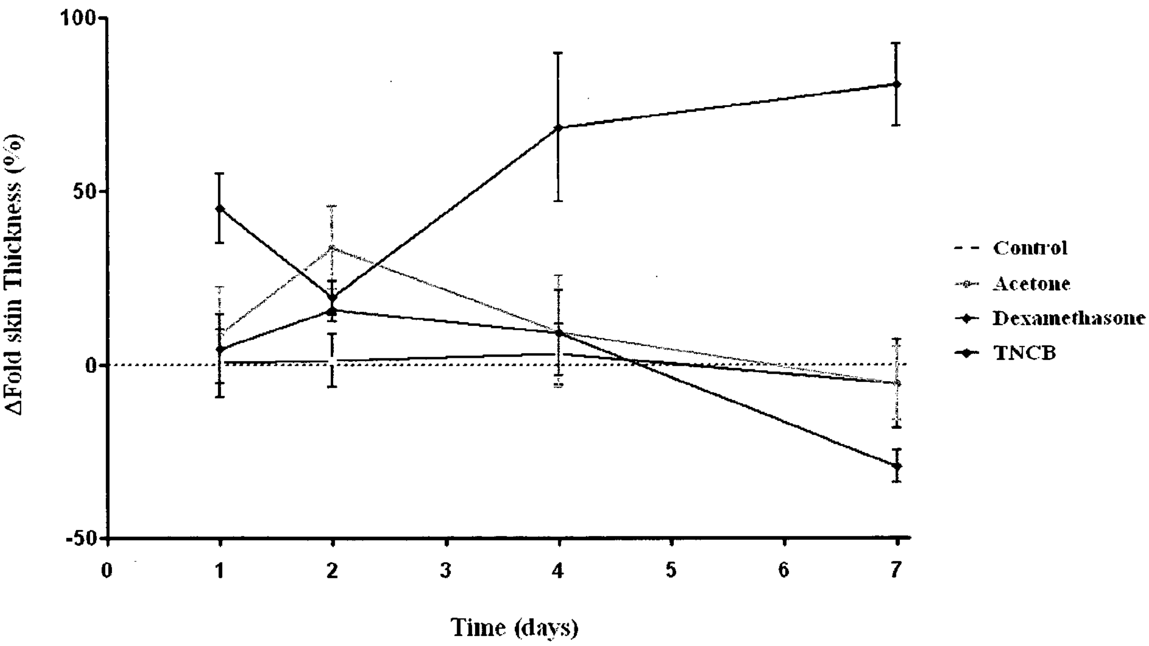
100
50
0
-50
ΔFold skin Thickness (%)
0
1
2
3
4
5
6
7
Time (days)
Control
Acetone
Dexamethasone
TNCB

FIGURE 5

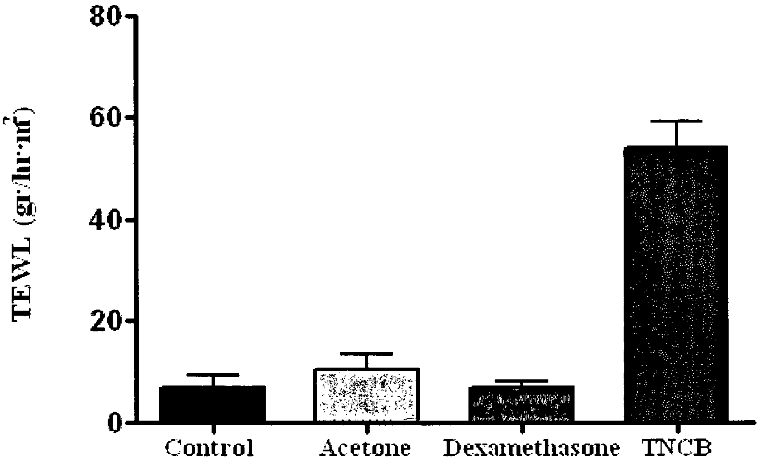


FIGURE 6

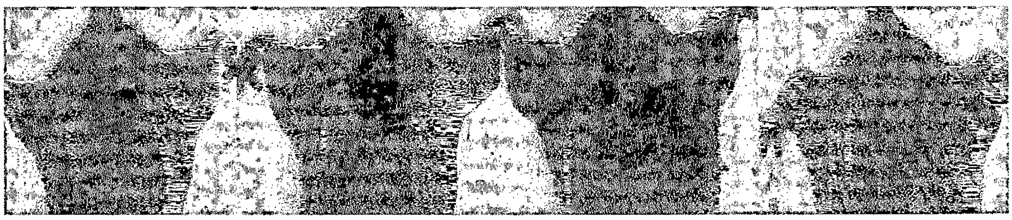

FIGURE 7

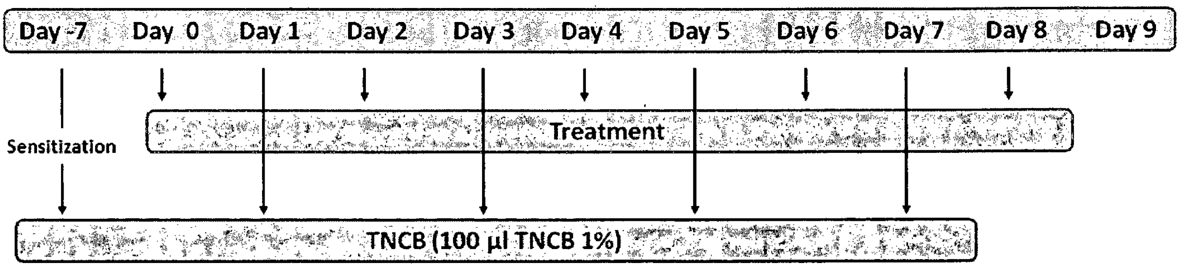

FIGURE 8

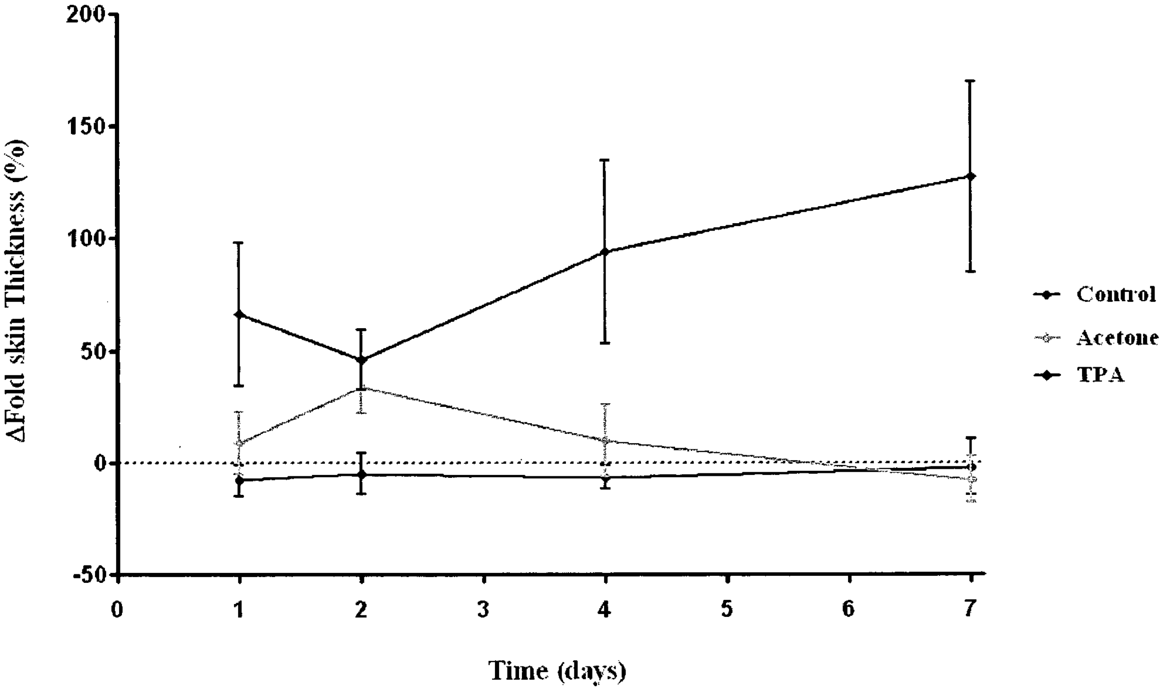


FIGURE 9

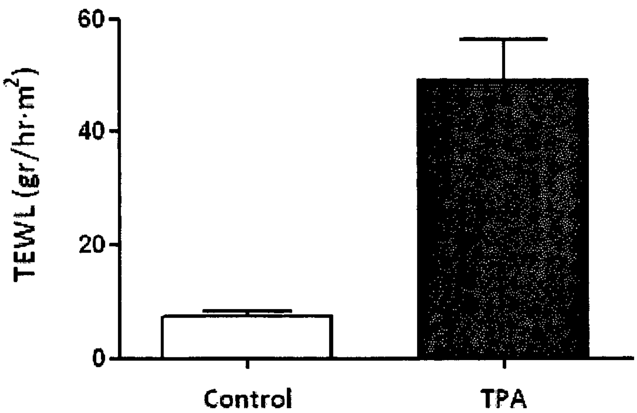

FIGURE 10

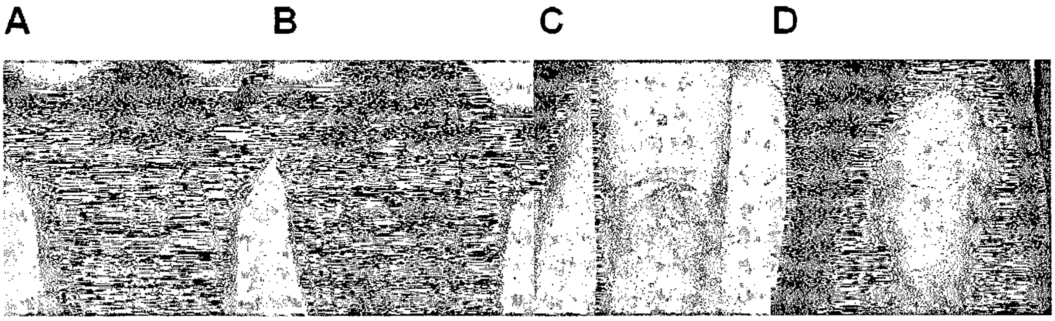


FIGURE 11

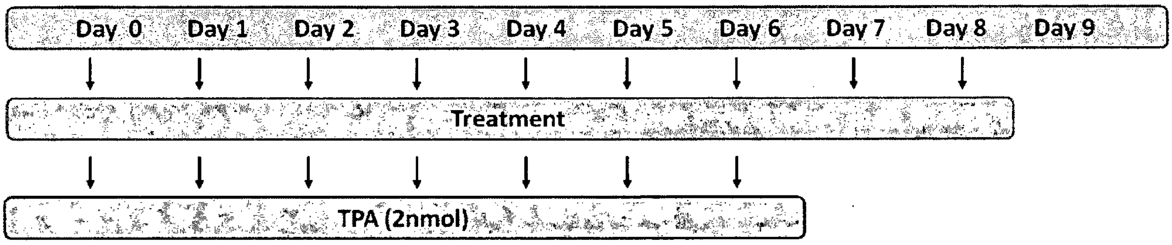


FIGURE 12

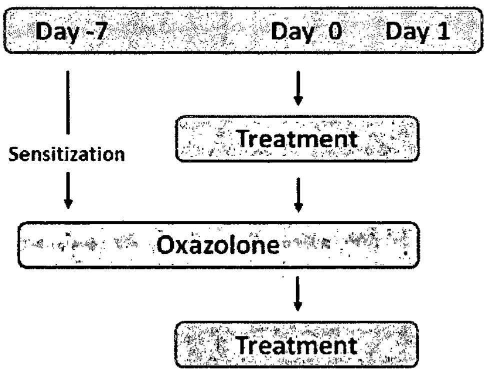

FIGURE 13

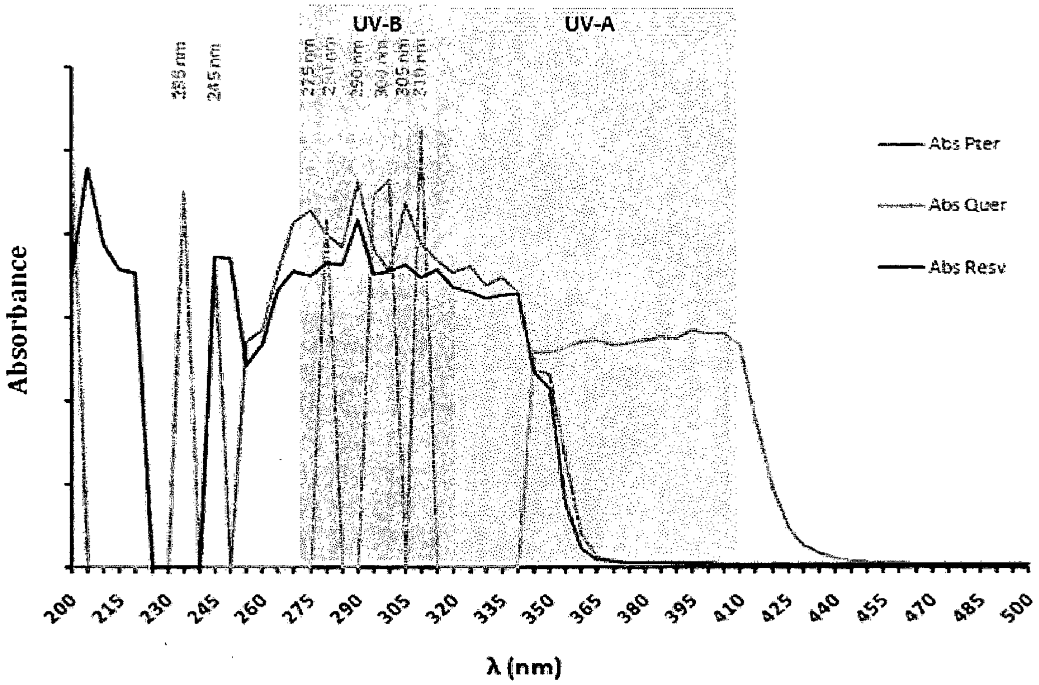


FIGURE 14

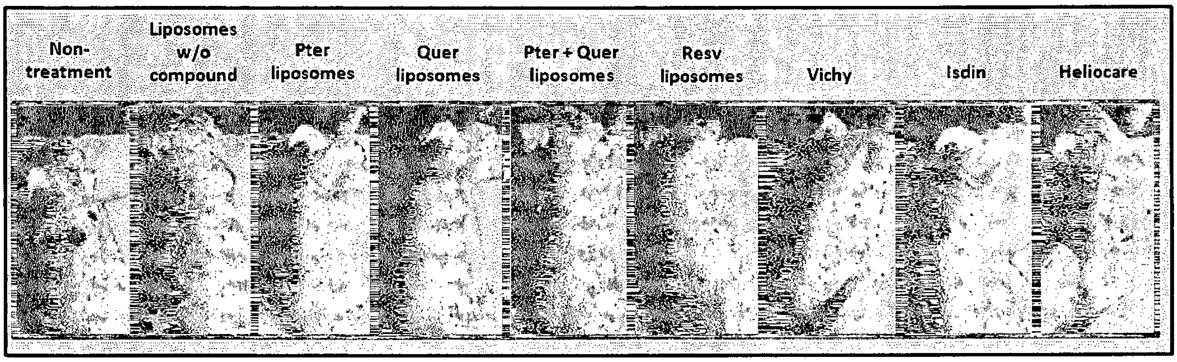

# FIGURE 15

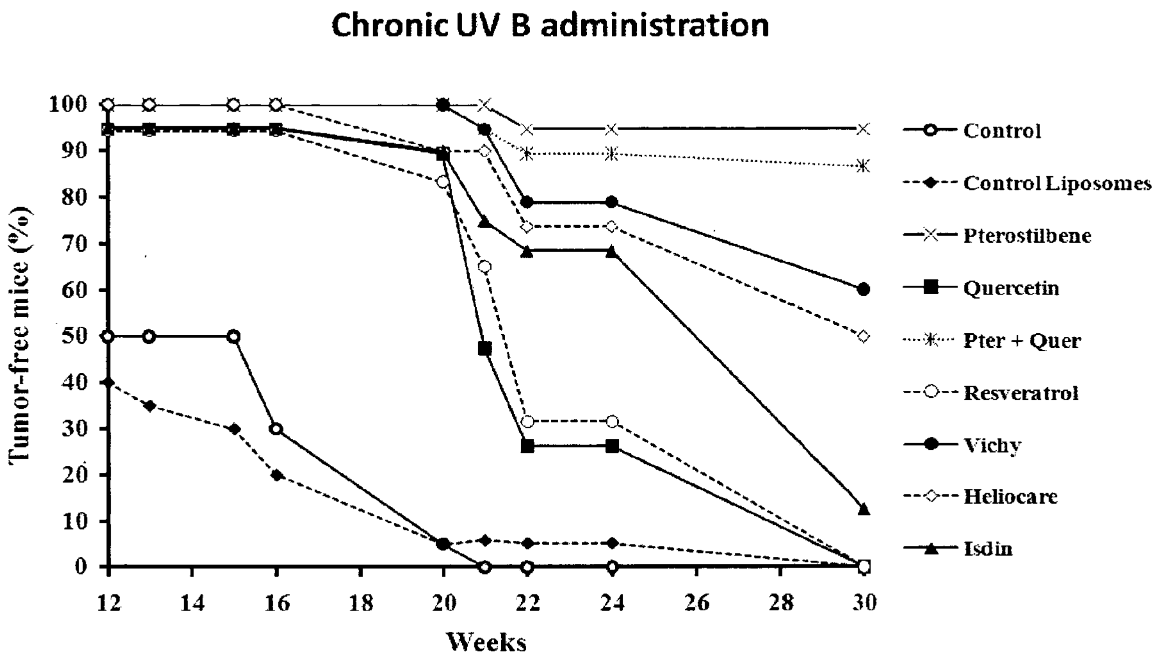
Chronic UV B administration
Tumor-free mice (%)
100
90
80
70
60
50
40
30
20
10
0
12
14
16
18
20
22
24
26
28
30
Weeks
Control
Control Liposomes
Pterostilbene
Quercetin
Pter + Quer
Resveratrol
Vichy
Heliocare
Isdin

# FIGURE 16

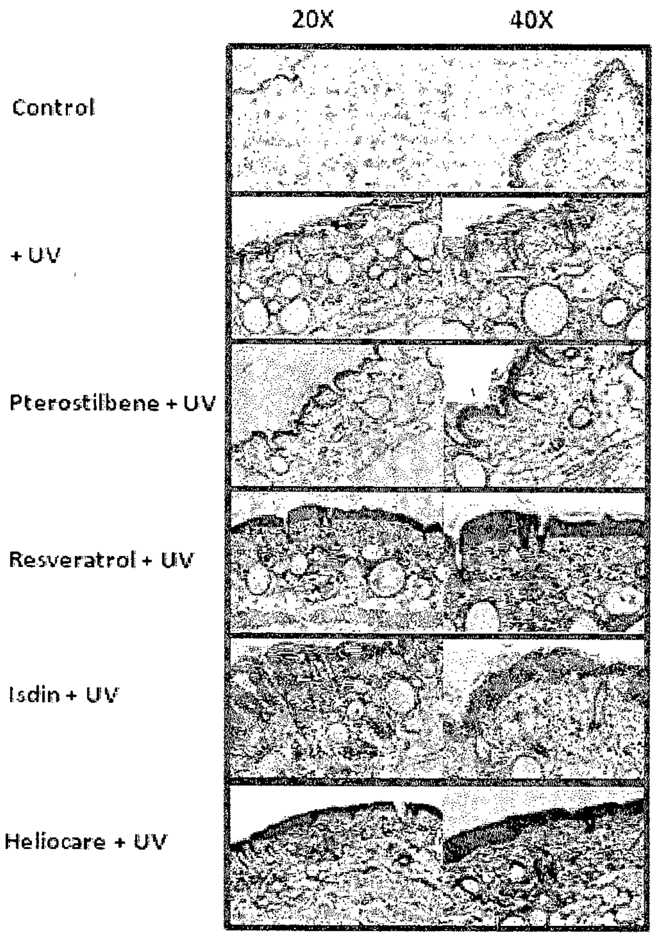
20X
40X
Control
+ UV
Pterostilbene + UV
Resveratrol + UV
Isdin + UV
Heliocare + UV

# FIGURE 17

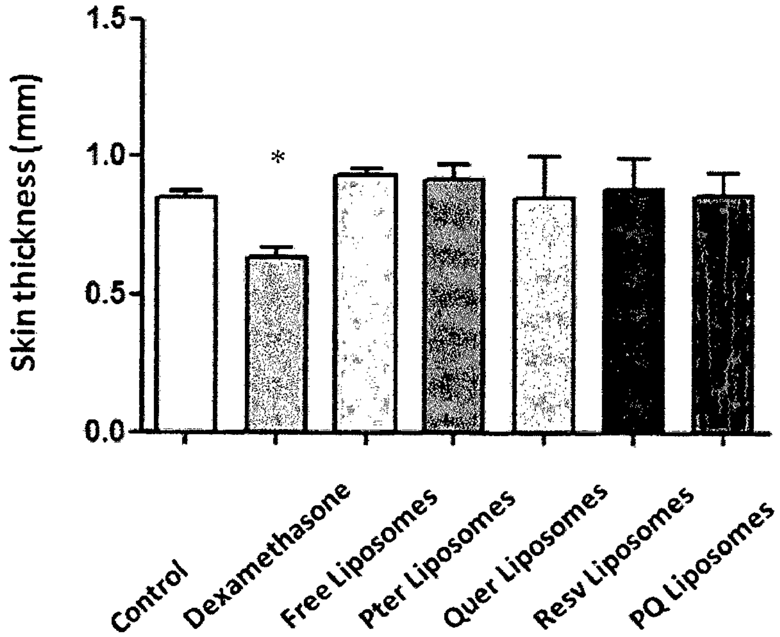

1.5
1.0
0.5
0.0
Skin thickness (mm)
*
Control
Dexamethasone
Free Liposomes
Pter Liposomes
Quer Liposomes
Resv Liposomes
PQ Liposomes

FIGURE 18

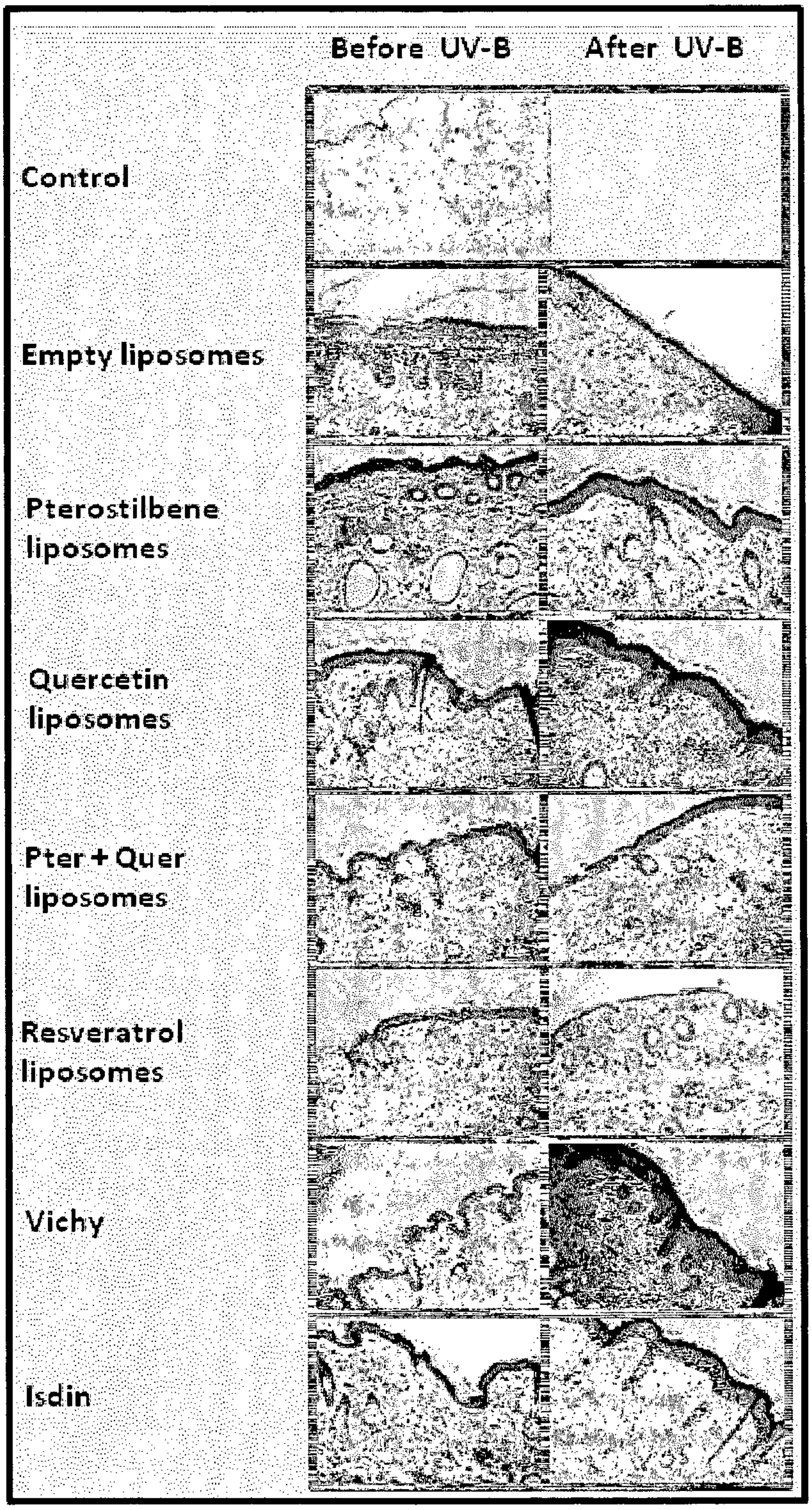
Before UV-B
After UV-B
Control
Empty liposomes
Pterostilbene liposomes
Quercetin liposomes
Pter + Quer liposomes
Resveratrol liposomes
Vichy
Isdin

## FIGURE 19

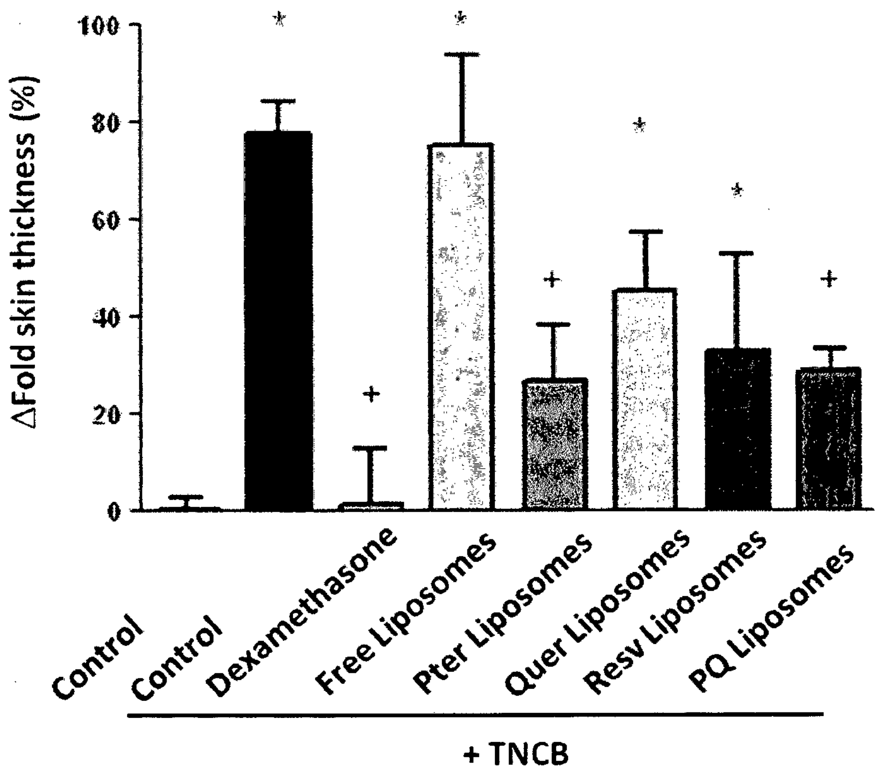


## FIGURE 20

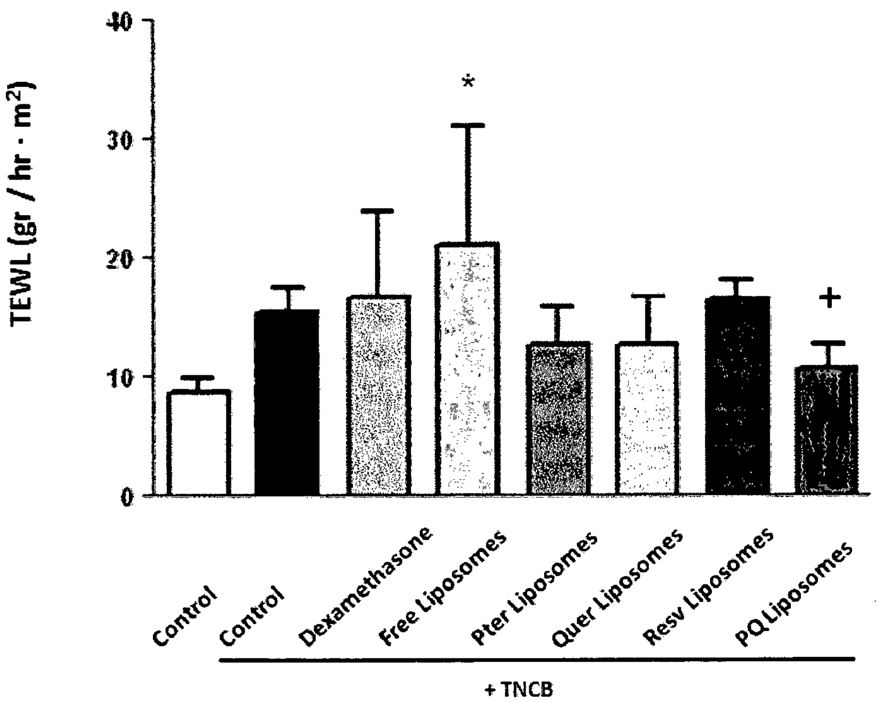

# FIGURE 21

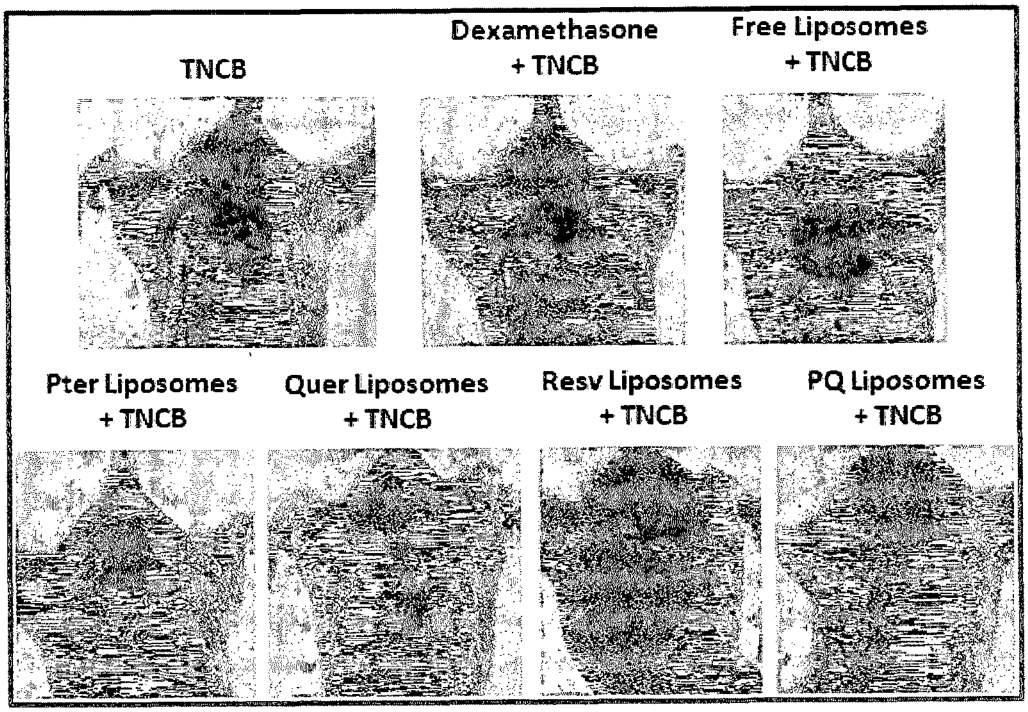
TNCB
Dexamethasone + TNCB
Free Liposomes + TNCB
Pter Liposomes + TNCB
Quer Liposomes + TNCB
Resv Liposomes + TNCB
PQ Liposomes + TNCB

FIGURE 22

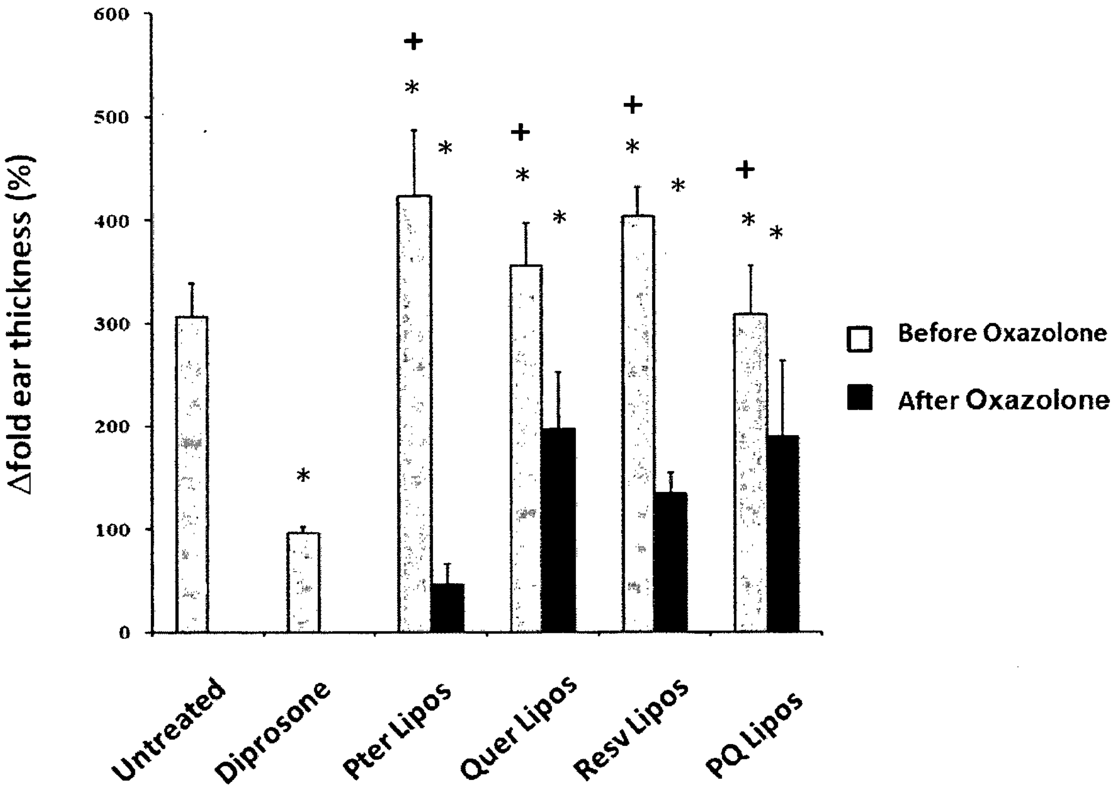
Δfold ear thickness (%)
600
500
400
300
200
100
0
Untreated
Diprosone
Pter Lipos
Quer Lipos
Resv Lipos
PQ Lipos
Before Oxazolone
After Oxazolone

FIGURE 23

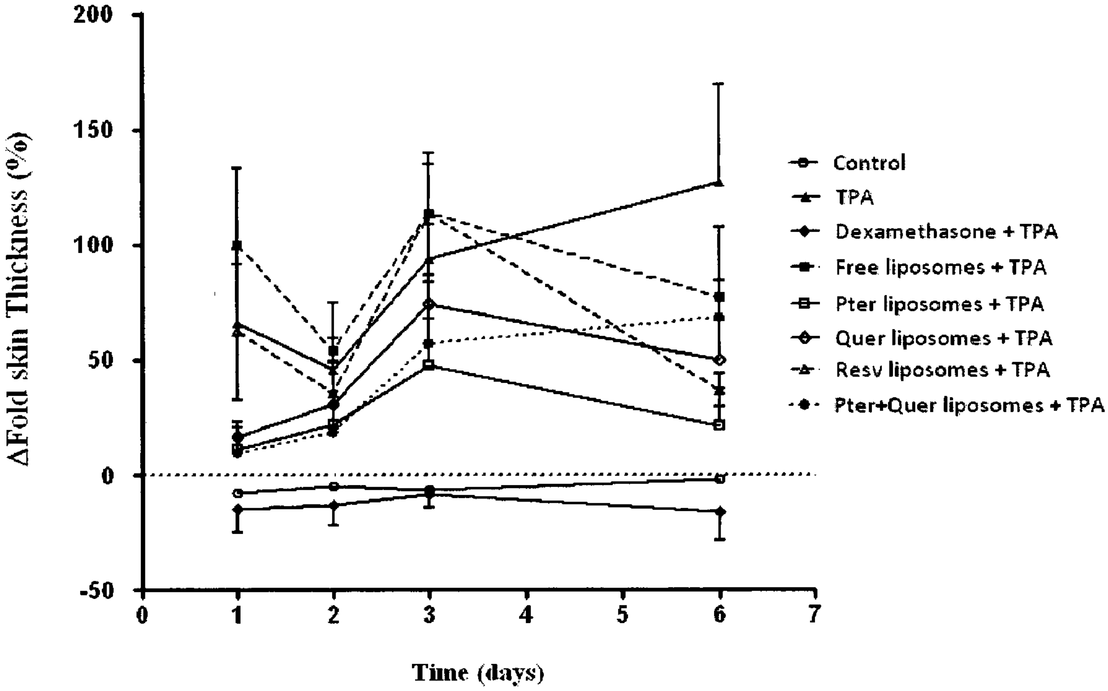
200
150
100
50
0
-50
0
1
2
3
4
5
6
7
ΔFold skin Thickness (%)
Time (days)
Control
TPA
Dexamethasone + TPA
Free liposomes + TPA
Pter liposomes + TPA
Quer liposomes + TPA
Resv liposomes + TPA
Pter+Quer liposomes + TPA

FIGURE 24

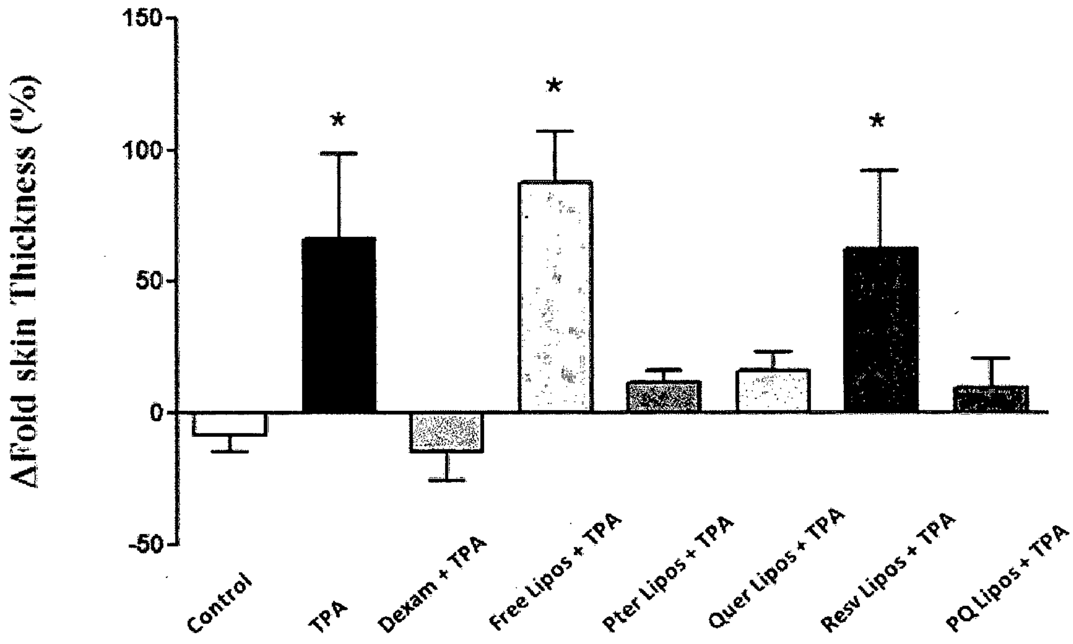


FIGURE 25

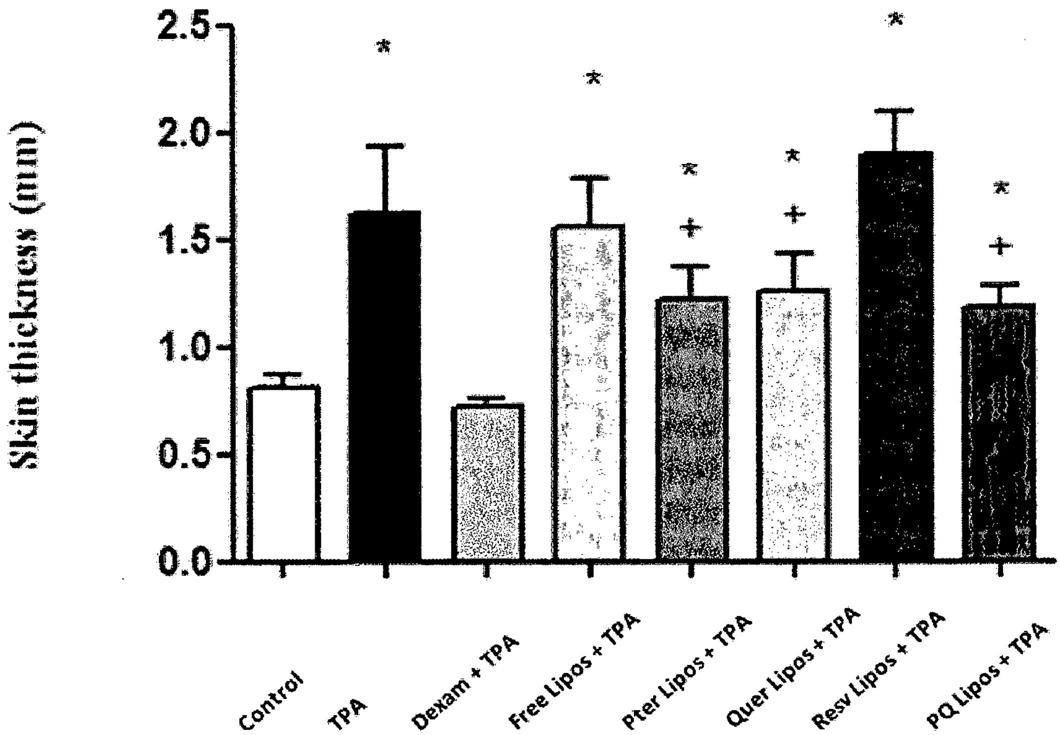

# FIGURE 26

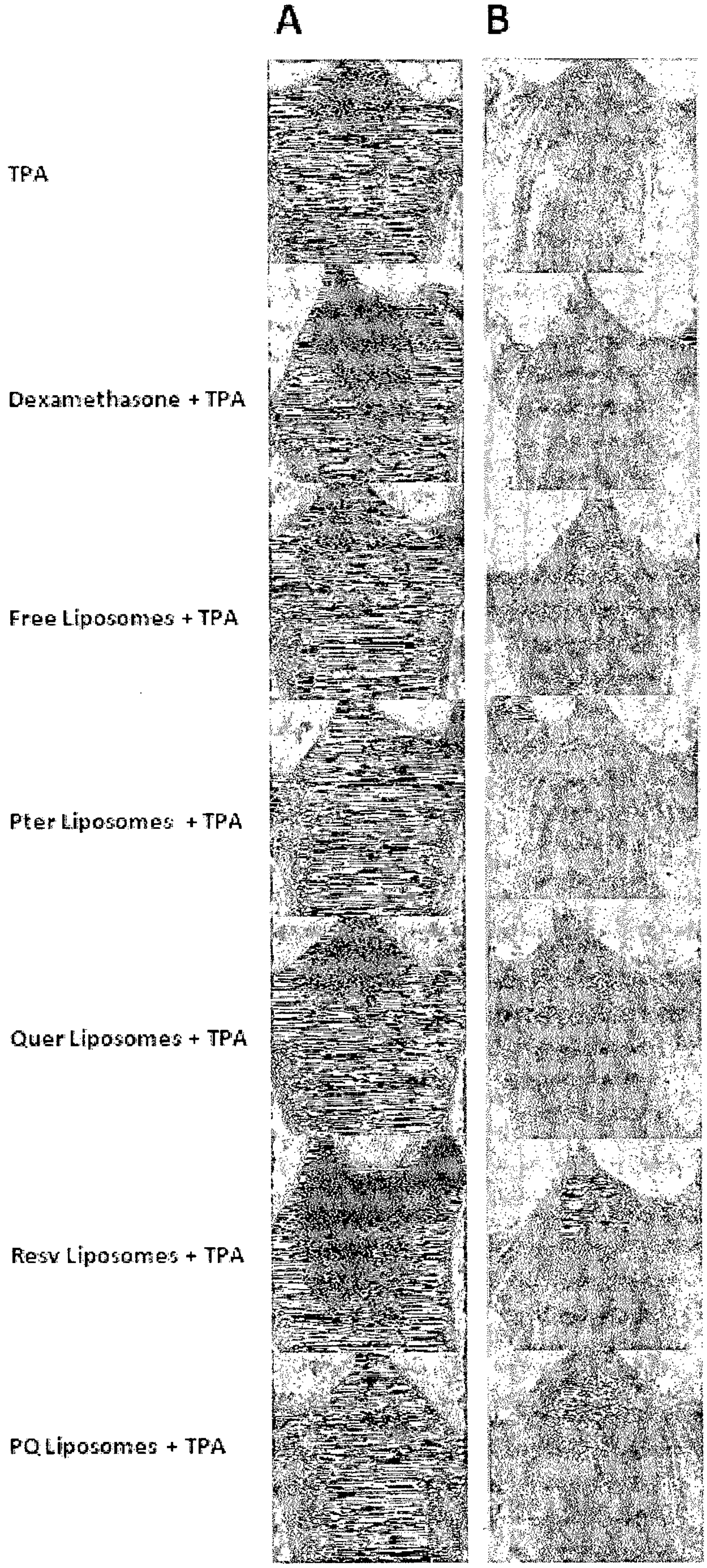
A
B
TPA
Dexamethasone + TPA
Free Liposomes + TPA
Pter Liposomes + TPA
Quer Liposomes + TPA
Resv Liposomes + TPA
PQ Liposomes + TPA

# FIGURE 27

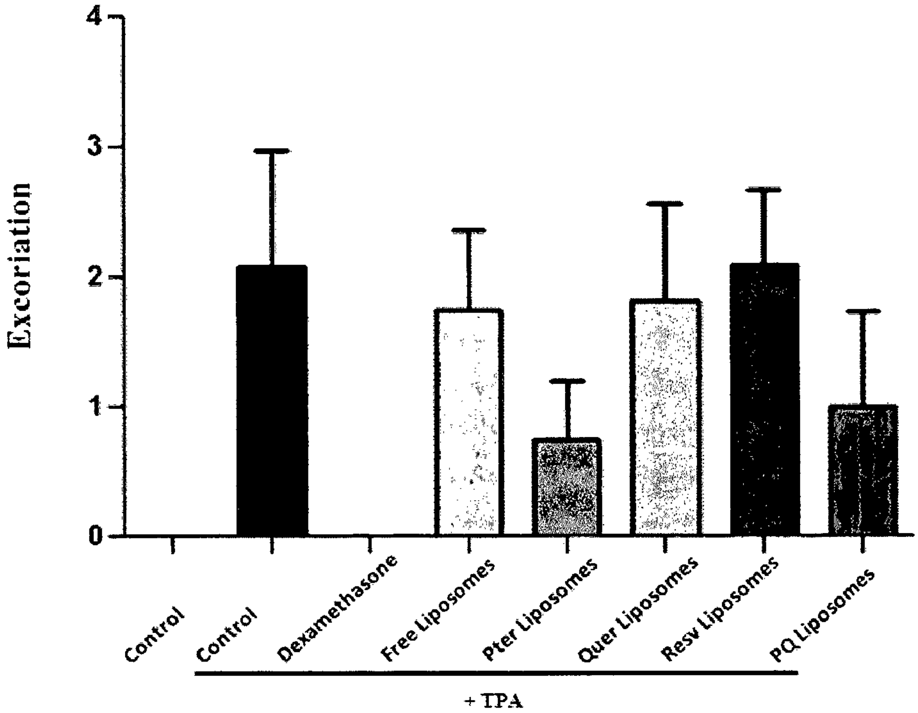
Excoriation
0
1
2
3
4
Control
Control
Dexamethasone
Free Liposomes
Pter Liposomes
Quer Liposomes
Resv Liposomes
PQ Liposomes
+ TPA

# FIGURE 28

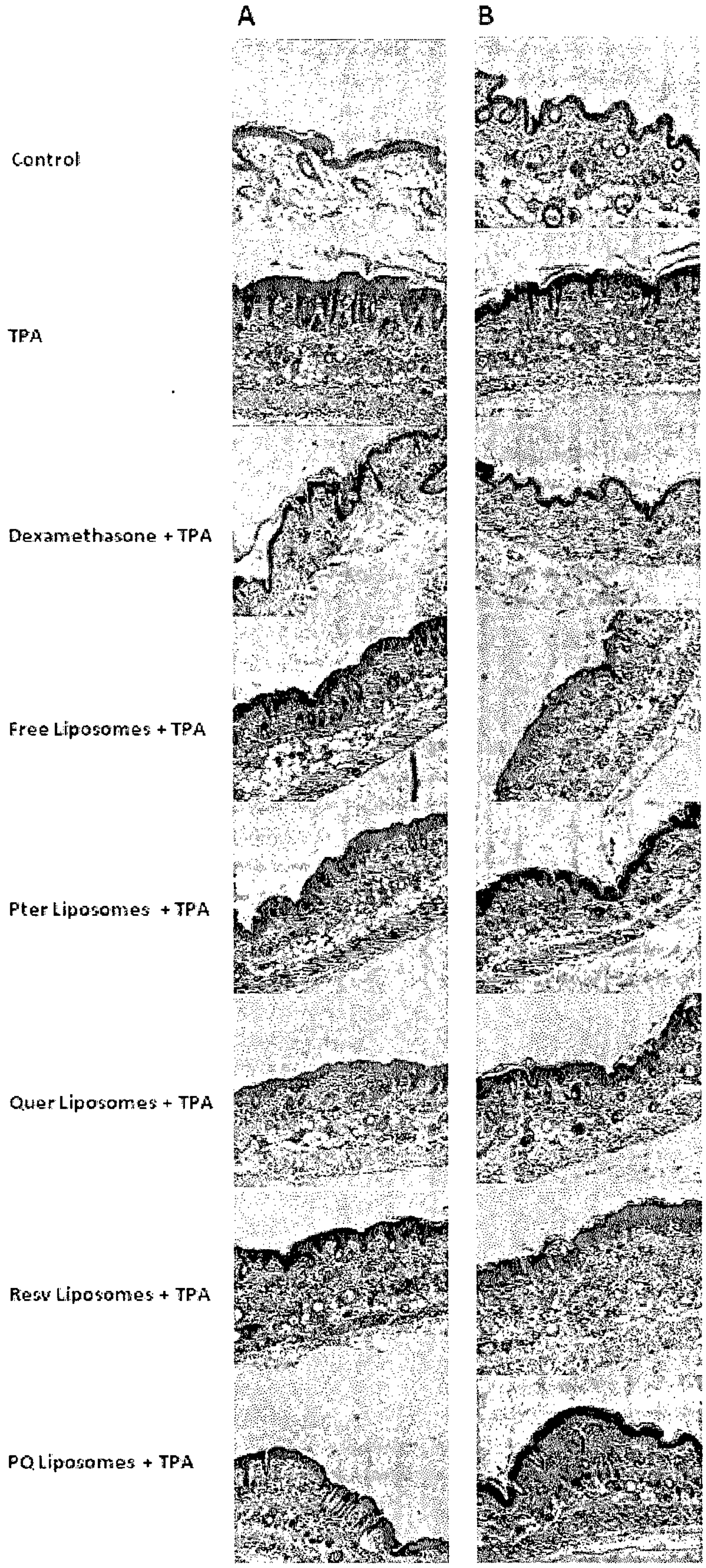
A
B
Control
TPA
Dexamethasone + TPA
Free Liposomes + TPA
Pter Liposomes + TPA
Quer Liposomes + TPA
Resv Liposomes + TPA
PQ Liposomes + TPA

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0143705 A2
- WO 2008067928 A


### Non-patent literature cited in the description


- **JANG M ; CAI L ; UDEANI GO ; SLOWING KV ; THOMAS CF, ; BEECHER CW ; FONG HH ; FARNSWORTH NR ; KINGHORN AD ; MEHTA RG.** *Science,* 1997, vol. 275 (5297), 218-20
- **ASENSI M ; MEDINA I ; ORTEGA A ; CARRETERO J ; BANO MC ; OBRADOR E ; ESTRELA JM.** *Free adic Biol Metal.,* 2002, vol. 33 (3), 387-98
- **FERRER P ; ASENSI M ; SEGARRA R ; ORTEGA A ; BENLLOCH M ; OBRADOR E ; VAREA MT ; ASENSIO G ; JORDA L ; ESTRELA JM.** *Neoplasia,* 2005, vol. 7 (1), 37-47
- **PRIEGO S ; FEDDI F ; FERRER P ; MENA S ; BENLLOCH M ; ORTEGA A ; CARRETERO J ; OBRADOR E ; ASENSI M ; ESTRELA JM.** *Mol Cancer Ther,* 2008, vol. 7 (10), 3330-42
- **RUBIA CASAGRANDE ; SANDRA R. GEORGETTI ; WALDICEU A. VERRI JR. ; DANIEL J. DORTA ; ANTÔNIO C. DOS SANTOS ; MARIA J. V. FONSECA.** *J. Photochem Photobiol B: Biology,* 2006, vol. 84, 21-27
- **ALENA SVOBODOVÁ ; JITKA PSOTOVÁ ; DANIELA WALTEROVÁ.** *Biomed. Papers,* 2003, vol. 147 (2), 137-145
- **MOAMMIR HASAN AZIZ ; SHANNON REAGAN-SHAW ; JIANQUIANG WU ; B. JACK LONGLEY ; NIHAL AHMAD.** *FASEB J.,* 2005, vol. 10 (1096), 1-18